(19) Europäisches Patentamt
European Patent Office
Office européen des brevets

(11) **EP 2 662 026 A1**

(12) **EUROPEAN PATENT APPLICATION**

(43) Date of publication:
**13.11.2013 Bulletin 2013/46**

(51) Int Cl.:
***A61B 8/06*** *(2006.01)* ***A61B 5/029*** *(2006.01)*

(21) Application number: **13166623.2**

(22) Date of filing: **06.05.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.05.2012 JP 2012106391**
**25.04.2013 JP 2013092007**

(71) Applicants:
- **Seiko Epson Corporation**
  **Tokyo 163 (JP)**
- **National Cerebral and Cardiovascular Center**
  **Suita-shi**
  **Osaka 565-8565 (JP)**

(72) Inventors:
- **Aoki, Mikio**
  **Nagano, 392-8502 (JP)**
- **Uemura, Kazunori**
  **Osaka, 565-8565 (JP)**
- **Sugimachi, Masaru**
  **Osaka, 565-8565 (JP)**

(74) Representative: **HOFFMANN EITLE**
**Patent- und Rechtsanwälte**
**Arabellastrasse 4**
**81925 München (DE)**

(54) **Cardiac output monitoring system and cardiac output measurement method**

(57) A processing device included in a cardiac output monitoring system includes an arterial diameter measurement ultrasonic probe that is attached to a neck of a subject, and measures the diameter of the carotid artery, and an arterial diameter measurement control section. A carotid artery diameter control section analyzes an ultrasonic echo to calculate the diameter of the artery (DOA), and outputs the diameter of the artery (DOA) to an ejection time/mean arterial pressure calculation section. The ejection time/mean arterial pressure calculation section calculates the ejection time (ET) from a temporal change in the diameter of the artery (DOA), and calculates the cardiac output. The temporal change waveform of the diameter of the artery (DOA) is clearer than the waveform of the blood flow velocity in the aorta or the ventricular outflow tract measured using an ultrasonic Doppler method, and the ejection time (ET) can be automatically calculated with high accuracy.

FIG. 1

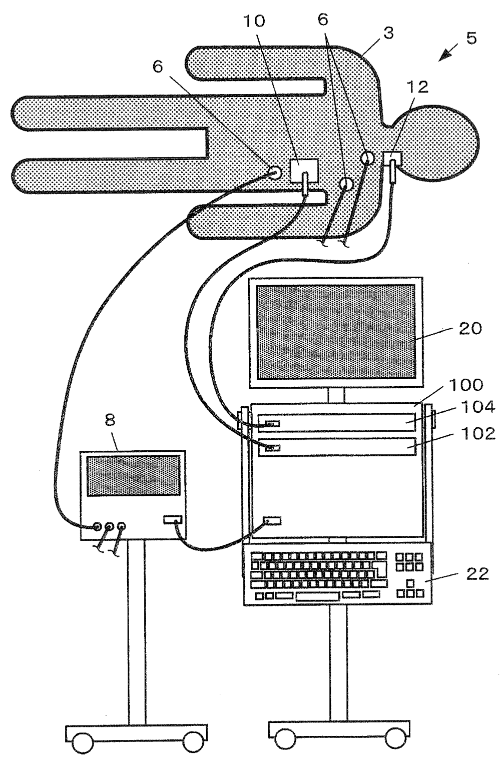




Printed by Jouve, 75001 PARIS (FR)

## Description

BACKGROUND

The cardiac output (CO) that indicates the volume of blood pumped out of the heart within 1 minute has been known as an index for evaluating the pumping function of the heart. For example, the cardiac output (CO) has been used in cardiac clinical practice together with biological information (e.g., electrocardiogram and blood pressure) in order to determine the state of the patient.

A technique that noninvasively measures the cardiac output (CO) has been widely studied. For example, JP-A-10-94528 discloses a technique that estimates the cardiac output (CO) from the radial artery pressure detected by a pressure wave sensor that is attached to the wrist of a patient, and displays the temporal change in the estimated value in graphical representation (horizontal axis: elapsed time).

However, a practical cardiac output (CO) measurement device that can be reliably used in medical practice has not been proposed.

Therefore, a manual method has been used in medical practice that measures the diameter of the ascending aorta and the flow velocity of blood that flows within the ascending aorta by utilizing an echocardiogram, and calculates the cardiac output (CO) from the measurement results. As illustrated in FIG. 20, a parasternal long-axis view 92 is obtained by M-mode echocardiography using a first transthoracic ultrasonic probe 90 to measure the cross-sectional area (CSA) of the aorta ($cm^2$), while the flow velocity (V) (cm/sec) in the left ventricular outflow tract within the systolic phase is measured from the cardiac apex in a continuous-wave Doppler mode using a second transthoracic ultrasonic probe 94. The velocity time integral (VTI) (cm) is then calculated, and the stroke volume (SV) ($cm^3$) is calculated by calculating a product of the cross-sectional area (CSA) and the velocity time integral (VTI). The cardiac output (CO) ($cm^3$/min or L/min) is calculated by calculating a product of the stroke volume (SV) and the heart rate (HR) (/min) that is measured separately.

When using the above manual measurement method, it is necessary to maintain the transthoracic ultrasonic probes 90 and 94 at a position and a posture appropriate for measuring the diameter of the artery and the flow velocity. Moreover, skill is required for the operator to find an appropriate position and posture. Therefore, the above measurement method has been mainly used to temporarily measure the cardiac output, and rarely used for continuous monitoring.

Moreover, a problem occurs when implementing the above method using a automated system. For example, a problem occurs when automatically calculating the velocity time integral (VTI) from the flow velocity data about the left ventricular outflow tract. Specifically, since the flow velocity signal in the diastolic phase obtained using the continuous-wave Doppler method contains noise (e.g., clutter sound due to opening and closing of the valve), the waveform is vague at or around the onset and end of ejection, and it is difficult to detect the ejection time (ET) from a flow velocity waveform 96 measured by the second transthoracic ultrasonic probe 94.

SUMMARY

According to one aspect of the invention, there is provided a cardiac output monitoring system comprising:

a flow velocity peak value detection section that detects a flow velocity peak value, the flow velocity peak value being a peak value of a flow velocity in a first artery or a ventricular outflow tract that has been measured from a body surface using a first sensor section;
an ejection time calculation section that calculates an ejection time from a temporal change in diameter of an artery, the diameter of the artery being a diameter of a second artery that has been measured from a body surface using a second sensor section;
a cross-sectional area estimation section that estimates a cross-sectional area of the first artery from the diameter of the second artery using a relationship between the diameter of the second artery and the cross-sectional area of the first artery that has been specified in advance;
a stroke volume calculation section that calculates a stroke volume using the flow velocity peak value, the ejection time, and the cross-sectional area; and
a cardiac output calculation section that calculates a cardiac output using the stroke volume and a given heart rate.

According to another aspect of the invention, there is provided a cardiac output monitoring system comprising:

a flow velocity peak value detection section that detects a flow velocity peak value, the flow velocity peak value being a peak value of a flow velocity in a first artery or a ventricular outflow tract that has been measured from a body surface using a first sensor section;
an ejection time calculation section that calculates an ejection time from a temporal change in arterial pressure, the arterial pressure being a pressure of a third artery using a third sensor section, the third artery being an artery that is palpable from a body surface;
a cross-sectional area estimation section that estimates a cross-sectional area of the first artery from the arterial pressure of the third artery using a relationship between the arterial pressure of the third artery and the cross-sectional area of the first artery that has been specified in advance;
a stroke volume calculation section that calculates a stroke volume using the flow velocity peak value, the ejection time, and the cross-sectional area; and
a cardiac output calculation section that calculates a cardiac output using the stroke volume and a given heart rate.

According to another aspect of the invention, there is provided a cardiac output monitoring system comprising:

a flow velocity peak value detection section that detects a flow velocity peak value, the flow velocity peak value being a peak value of a flow velocity in a first artery or a ventricular outflow tract that has been measured from a body surface using a first sensor section;
a cross-sectional area estimation section that estimates a cross-sectional area of the first artery from a diameter of a second artery using a relationship between the diameter of the second artery and the cross-sectional area of the first artery that has been specified in advance, the diameter of the second artery being a diameter that has been measured from a body surface using a second sensor section;
an ejection time calculation section that calculates an ejection time from a temporal change in arterial pressure, the arterial pressure being a pressure of a third artery using a third sensor section, the third artery being an artery that is palpable from a body surface;
a stroke volume calculation section that calculates a stroke volume using the flow velocity peak value, the ejection time, and the cross-sectional area; and
a cardiac output calculation section that calculates a cardiac output using the stroke volume and a given heart rate.

According to another aspect of the invention, there is provided a cardiac output measurement method comprising:

detecting a flow velocity peak value, the flow velocity peak value being a peak value of a flow velocity in a first artery or a ventricular outflow tract that has been measured from a body surface using a first sensor section;
calculating an ejection time from a temporal change in diameter of an artery, the diameter of the artery being a diameter of a second artery that has been measured from a body surface using a second sensor section;
estimating a cross-sectional area of the first artery from the diameter of the second artery using a relationship between the diameter of the second artery and the cross-sectional area of the first artery that has been specified in advance;
calculating a stroke volume using the flow velocity peak value, the ejection time, and the cross-sectional area; and
calculating a cardiac output using the stroke volume and a given heart rate.

According to another aspect of the invention, there is provided a cardiac output measurement method comprising:

detecting a flow velocity peak value, the flow velocity peak value being a peak value of a flow velocity in a first artery or a ventricular outflow tract that has been measured from a body surface using a first sensor section;
calculating an ejection time from a temporal change in arterial pressure, the arterial pressure being a pressure of a third artery using a third sensor section, the third artery being an artery that is palpable from a body surface;
estimating a cross-sectional area of the first artery from the arterial pressure of the third artery using a relationship between the arterial pressure of the third artery and the cross-sectional area of the first artery that has been specified in advance;
calculating a stroke volume using the flow velocity peak value, the ejection time, and the cross-sectional area; and
calculating a cardiac output using the stroke volume and a given heart rate.

According to another aspect of the invention, there is provided a cardiac output measurement method comprising:

detecting a flow velocity peak value, the flow velocity peak value being a peak value of a flow velocity in a first artery or a ventricular outflow tract that has been measured from a body surface using a first sensor section;
estimating a cross-sectional area of the first artery from a diameter of a second artery using a relationship between the diameter of the second artery and the cross-sectional area of the first artery that has been specified in advance, the diameter of the second artery being a diameter that has been measured from a body surface using a second sensor section;
calculating an ejection time from a temporal change in arterial pressure, the arterial pressure being a pressure of a third artery using a third sensor section, the third artery being an artery that is palpable from a body surface;
calculating a stroke volume using the flow velocity peak value, the ejection time, and the cross-sectional area; and
calculating a cardiac output using the stroke volume and a given heart rate.

BRIEF DESCRIPTION OF THE DRAWINGS

FIG. 1 is a view illustrating a system configuration example of a cardiac output monitoring system according to a first embodiment.
FIG. 2 is a functional block diagram illustrating a functional configuration example according to the first embodiment.
FIG. 3 is a view illustrating detection of a flow velocity peak value according to the first embodiment.
FIG. 4 is a view illustrating calculation of an ejection time and a mean arterial pressure according to the first embodiment.
FIG. 5 is a view illustrating estimation of a cross-sectional area of the aorta according to the first embodiment.
FIG. 6 is a view illustrating calculation of a cardiac output according to the first embodiment.
FIG. 7 is a flowchart illustrating the flow of a process performed by the cardiac output monitoring system according to the first embodiment.
FIG. 8 is a functional block diagram illustrating a functional configuration example according to a second embodiment.
FIG. 9 is a view illustrating estimation of a cross-sectional area of the aorta according to the second embodiment.
FIG. 10 is a view illustrating a system configuration example of a cardiac output monitoring system according to a third embodiment.
FIG. 11 is a functional block diagram illustrating a functional configuration example according to the third embodiment.
FIG. 12 is a view illustrating calculation of an ejection time and a mean arterial pressure according to the third embodiment.
FIG. 13 is a view illustrating a system configuration example of a cardiac output monitoring system according to a fourth embodiment.
FIG. 14 is a functional block diagram illustrating a functional configuration example according to the fourth embodiment.
FIG. 15 is a graph showing the cardiac output measurement results obtained using the cardiac output monitoring system according to the third embodiment.
FIG. 16 is a graph showing the cardiac output measurement results obtained using the cardiac output monitoring system according to the third embodiment.
FIG. 17 is a view illustrating a modification of the functional configuration of a flow velocity measurement module.
FIG. 18 is a view illustrating a modification of the functional configuration of an arterial diameter measurement module.
FIG. 19 is a flowchart illustrating the flow of a process performed by the cardiac output monitoring system according to the first embodiment.
FIG. 20 is a view illustrating a related-art cardiac output measurement method.

DESCRIPTION OF EXEMPLARY EMBODIMENTS

Several embodiments of the invention may make it possible to implement a novel technique that noninvasively and continuously measures the cardiac output.
According to one embodiment of the invention, there is provided a cardiac output monitoring system comprising:

a flow velocity peak value detection section that detects a flow velocity peak value, the flow velocity peak value being a peak value of a flow velocity in a first artery or a ventricular outflow tract that has been measured from a body surface using a first sensor section;
an ejection time calculation section that calculates an ejection time from a temporal change in diameter of an artery, the diameter of the artery being a diameter of a second artery that has been measured from a body surface using a second sensor section;
a cross-sectional area estimation section that estimates a cross-sectional area of the first artery from the diameter of the second artery using a relationship between the diameter of the second artery and the cross-sectional area of the first artery that has been specified in advance;
a stroke volume calculation section that calculates a stroke volume using the flow velocity peak value, the ejection time, and the cross-sectional area; and
a cardiac output calculation section that calculates a cardiac output using the stroke volume and a given heart rate.

According to another embodiment of the invention, there is provided a cardiac output monitoring system comprising:

a flow velocity peak value detection section that detects a flow velocity peak value, the flow velocity peak value being a peak value of a flow velocity in a first artery or a ventricular outflow tract that has been measured from a body surface using a first sensor section;
an ejection time calculation section that calculates an ejection time from a temporal change in arterial pressure, the arterial pressure being a pressure of a third artery using a third sensor section, the third artery being an artery that is palpable from a body surface;
a cross-sectional area estimation section that estimates a cross-sectional area of the first artery from the arterial pressure of the third artery using a relationship between the arterial pressure of the third artery and the cross-sectional area of the first artery that has been specified in advance;
a stroke volume calculation section that calculates a stroke volume using the flow velocity peak value, the ejection time, and the cross-sectional area; and
a cardiac output calculation section that calculates a cardiac output using the stroke volume and a given heart rate.

According to another embodiment of the invention, there is provided a cardiac output monitoring system comprising:

a flow velocity peak value detection section that detects a flow velocity peak value, the flow velocity peak value being a peak value of a flow velocity in a first artery or a ventricular outflow tract that has been measured from a body surface using a first sensor section;
a cross-sectional area estimation section that estimates a cross-sectional area of the first artery from a diameter of a second artery using a relationship between the diameter of the second artery and the cross-sectional area of the first artery that has been specified in advance, the diameter of the second artery being a diameter that has been measured from a body surface using a second sensor section;
an ejection time calculation section that calculates an ejection time from a temporal change in arterial pressure, the arterial pressure being a pressure of a third artery using a third sensor section, the third artery being an artery that is palpable from a body surface;
a stroke volume calculation section that calculates a stroke volume using the flow velocity peak value, the ejection time, and the cross-sectional area; and
a cardiac output calculation section that calculates a cardiac output using the stroke volume and a given heart rate.

According to the above configuration, since the ejection time can be calculated using a value other than the flow velocity in the ventricular outflow tract, the cardiac output can be calculated more accurately based on the velocity time integral and the cross-sectional area without being affected by a noise component contained in the flow velocity waveform. This makes it possible to noninvasively and continuously measure the cardiac output.
In the cardiac output monitoring system,
the first sensor section may be a sensor that transmits and receives an ultrasonic beam,
the cardiac output monitoring system may further comprise a first ultrasonic beam control section that controls either or both of ultrasonic beam focusing and ultrasonic beam scanning performed by the first sensor section so that (a) a temporal change waveform of the flow velocity based on a detection signal of the first sensor section satisfies a given clearness condition or (b) the flow velocity peak value detected by the flow velocity peak value detection section becomes a maximum, or (c) the temporal change waveform of the flow velocity based on the detection signal of the first sensor section satisfies the given clearness condition and the flow velocity peak value detected by the flow velocity peak value detection section becomes a maximum.
This makes it possible to accurately and stably measure the flow velocity peak value.
In the cardiac output monitoring system,
the second sensor section may be a sensor that transmits and receives an ultrasonic beam,
the cardiac output monitoring system may further comprise a second ultrasonic beam control section that controls either or both of ultrasonic beam focusing and ultrasonic beam scanning performed by the second sensor section so that (a) a major axis length and a minor axis length of a cross section of the second artery satisfy a given equality condition or (b) the cross section satisfies a given circularity condition, or (c) the major axis length and the minor axis length of the cross section of the second artery satisfy the given equality condition and the cross section satisfies the given circularity condition.

This makes it possible to accurately and stably measure the diameter of the artery.

In the cardiac output monitoring system,
the first sensor section may be a thin and flat ultrasonic probe that is attached to a surface of a chest wall of a subject, and configured so that a focus of an ultrasonic beam can be changed in three-dimensional directions.

This makes it possible to fix the ultrasonic probe. Since the ultrasonic probe is thin and flat, the ultrasonic probe can be more easily attached to the subject, and it is possible to suppress a change in position of the ultrasonic probe due to a change in posture of the subject.

The cardiac output monitoring system may further comprise:

a display control section that updates and displays the cardiac output each time the cardiac output has been calculated by the cardiac output calculation section; and
an alarm control section that gives a given alarm when the cardiac output calculated by the cardiac output calculation section has satisfied a given alarm condition.

This makes it possible to always display the monitored value. It is also possible to call attention when the cardiac output is abnormal by automatically giving an alarm.

According to another embodiment of the invention, there is provided a cardiac output measurement method comprising:

detecting a flow velocity peak value, the flow velocity peak value being a peak value of a flow velocity in a first artery or a ventricular outflow tract that has been measured from a body surface using a first sensor section;
calculating an ejection time from a temporal change in diameter of an artery, the diameter of the artery being a diameter of a second artery that has been measured from a body surface using a second sensor section;
estimating a cross-sectional area of the first artery from the diameter of the second artery using a relationship between the diameter of the second artery and the cross-sectional area of the first artery that has been specified in advance;
calculating a stroke volume using the flow velocity peak value, the ejection time, and the cross-sectional area; and
calculating a cardiac output using the stroke volume and a given heart rate.

According to another embodiment of the invention, there is provided a cardiac output measurement method comprising:

detecting a flow velocity peak value, the flow velocity peak value being a peak value of a flow velocity in a first artery or a ventricular outflow tract that has been measured from a body surface using a first sensor section;
calculating an ejection time from a temporal change in arterial pressure, the arterial pressure being a pressure of a third artery using a third sensor section, the third artery being an artery that is palpable from a body surface;
estimating a cross-sectional area of the first artery from the arterial pressure of the third artery using a relationship between the arterial pressure of the third artery and the cross-sectional area of the first artery that has been specified in advance;
calculating a stroke volume using the flow velocity peak value, the ejection time, and the cross-sectional area; and
calculating a cardiac output using the stroke volume and a given heart rate.

According to another embodiment of the invention, there is provided a cardiac output measurement method comprising:

detecting a flow velocity peak value, the flow velocity peak value being a peak value of a flow velocity in a first artery or a ventricular outflow tract that has been measured from a body surface using a first sensor section;
estimating a cross-sectional area of the first artery from a diameter of a second artery using a relationship between the diameter of the second artery and the cross-sectional area of the first artery that has been specified in advance, the diameter of the second artery being a diameter that has been measured from a body surface using a second sensor section;
calculating an ejection time from a temporal change in arterial pressure, the arterial pressure being a pressure of a third artery using a third sensor section, the third artery being an artery that is palpable from a body surface;
calculating a stroke volume using the flow velocity peak value, the ejection time, and the cross-sectional area; and
calculating a cardiac output using the stroke volume and a given heart rate.

In the cardiac output measurement method,
the estimation of the cross-sectional area may include estimating the cross-sectional area using an equation (1),

$$\text{cross-sectional area}=A\times DOA^2+B\times DOA+C \qquad (1)$$

where, DOA is the diameter of the second artery indicated by a detection signal of the second sensor section, and A, B, and C are constants set in advance, and
the calculating of the stroke volume may include calculating the stroke volume using an equation (2),

$$\text{stroke volume}=(Vpeak\times ET/2)\times CSA \qquad (2)$$

where, Vpeak is the detected flow velocity peak value, ET is the calculated ejection time, and CSA is the estimated cross-sectional area.
In the cardiac output measurement method,
the estimation of the cross-sectional area may include estimating the cross-sectional area using an equation (3),

$$\text{cross-sectional area}=E\times\{0.5+(1/\pi)\times\arctan[(AP-F)/G]\} \qquad (3)$$

where, arctan is an arc tangent function, AP is the arterial pressure of the third artery indicated by a detection signal of the third sensor section, and E, F, and G are constants set in advance, and
the calculation of the stroke volume may include calculating the stroke volume using an equation (4),

$$\text{stroke volume}=(Vpeak\times ET/2)\times CSA \qquad (4)$$

where, Vpeak is the detected flow velocity peak value, ET is the calculated ejection time, and CSA is the estimated cross-sectional area.
Exemplary embodiments of the invention are described below with reference to the drawings. Note that the invention is not limited to the following exemplary embodiments.

First embodiment

A first embodiment to which the invention is applied is described below.
FIG. 1 is a view illustrating a system configuration example of a cardiac output monitoring system 5 according to the first embodiment. The cardiac output monitoring system 5 according to the first embodiment includes (1) an electrocardiography module 8 that continuously generates and outputs an electrocardiogram waveform (ECG waveform) that is obtained through electrodes 6 attached to the surface of the chest of a subject 3, and indicates a temporal change in electrical activity of the heart, (2) a transthoracic ultrasonic probe 10 that is a first sensor section that measures the blood flow velocity in a first artery or the ventricular outflow tract from the body surface of the subject 3, (3) an arterial diameter measurement ultrasonic probe 12 that is a second sensor section that measures the diameter of a second artery from the body surface of the subject 3, (4) a processing device 100 that continuously calculates and outputs the cardiac output based on the data measured by the electrocardiography module 8, the transthoracic ultrasonic probe 10, and the arterial diameter measurement ultrasonic probe 12, (5) a video monitor 20, and (6) a keyboard 22.
The transthoracic ultrasonic probe 10 is a thin and flat pad-type ultrasonic probe that can be attached to the chest or the like of the subject 3, the transthoracic ultrasonic probe 10 implementing ultrasonic measurement using a continuous-wave Doppler method, and including a thin-film piezoelectric diaphragm-type ultrasonic transducer that generates and emits measurement ultrasonic waves, and converts reflected waves (ultrasonic echo) from the subject 3 into an electrical signal. In the first embodiment, the transthoracic ultrasonic probe 10 is attached to the surface of the left side of the chest of the subject 3 at a position selected to be appropriate for measuring the blood flow velocity in the ascending aorta or the ventricular outflow tract, for example.
The arterial diameter measurement ultrasonic probe 12 is a thin and flat pad-type ultrasonic probe that can be attached to the neck or the like of the subject 3, the arterial diameter measurement ultrasonic probe 12 including a thin-film piezoelectric diaphragm-type ultrasonic transducer that generates and emits measurement ultrasonic waves, and converts reflected waves (ultrasonic echo) from the subject 3 into an electrical signal. In the first embodiment, the arterial diameter measurement ultrasonic probe 12 is attached to the neck of the subject 3, for example. Note that the arterial diameter measurement ultrasonic probe 12 may be attached at a position corresponding to an artery other than the carotid artery.

Since the transthoracic ultrasonic probe 10 and the arterial diameter measurement ultrasonic probe 12 are implemented by a thin and flat pad-type ultrasonic probe that can be attached to the body of the subject 3, it is unnecessary for the operator to hold the ultrasonic probe during monitoring while adjusting the position of the ultrasonic probe, differing from a related-art transthoracic ultrasonic probe. Moreover, it is unnecessary to provide an auxiliary device (e.g., arm) for preventing or correcting a shift in position, and the cardiac output can be continuously measured.

The video monitor 20 is an image display device, and is implemented by a flat panel display or a touch panel display. The video monitor 20 may appropriately include a speaker.

The keyboard 22 is a means that allows the operator to input an operation. In the example illustrated in FIG. 1, the keyboard 22 is supported by a swing arm so that the keyboard 22 can swing, and is optionally raised forward. Note that the keyboard 22 may be integrated with the processing device 100, or may be implemented by a touch panel that is used as the video monitor 20. An additional operation input device such as a mouse or a trackpad may also be provided.

The processing device 100 is implemented by a microprocessor (e.g., central processing unit (CPU), graphics processing unit (GPU), or digital signal processor (DSP)), an application-specific integrated circuit (ASIC), an electronic circuit, an information storage medium such as an IC memory (e.g., VRAM, RAM, or ROM) or a hard disk, an interface IC that implements data transfer to the outside, a connection terminal, a power supply circuit, and the like.

The processing device 100 implements a flow velocity measurement module 102 that controls transmission and reception of ultrasonic waves by the transthoracic ultrasonic probe 10, and calculates the blood flow velocity from the received ultrasonic echo. The processing device 100 also implements an arterial diameter measurement module 104 that controls transmission and reception of ultrasonic waves by the arterial diameter measurement ultrasonic probe 12, and calculates the diameter of the artery from the received ultrasonic echo.

The processing device 100 calculates the cardiac output (CO) by performing a calculation process in accordance with a given program, and successively displays the calculation result on the video monitor 20 (i.e., successively updates the calculation result displayed on the video monitor 20). Specifically, the processing device 100, the video monitor 20, and the keyboard 22 implement a computer that continuously monitors the cardiac output (CO).

Note that FIG. 1 schematically illustrates the number and the positions of the electrodes 6 (i.e., the number and the positions of the electrodes 6 do not necessarily reflect the actual situation). The processing device 100 may appropriately include a communication device for implementing data communication with an external computer, a device that reads/writes data from/into a removable information storage medium, and the like.

Functional block diagram

FIG. 2 is a functional block diagram illustrating a functional configuration example according to the first embodiment.

The processing device 100 includes a heart rate calculation section 110, a flow velocity measurement control section 112, an arterial diameter measurement control section 114, a flow velocity peak value detection section 120, an ejection time/mean arterial pressure calculation section 122, a cross-sectional area estimation section 124, a cardiac output calculation section 130, a display control section 140, an alarm control section 142, and a storage section 150.

The heart rate calculation section 110 measures the time between the adjacent peaks of the QRS complex of the electrocardiogram waveform data output from the electrocardiography module 8 to calculate the heart rate (HR). The heart rate calculation section 110 may be implemented by an electronic circuit (e.g., DSP or ASIC), or may be implemented by a calculation process performed by a CPU or the like. Note that the electrocardiography module 8 may include a heart rate calculation circuit, and output the heart rate together with the electrocardiogram waveform data.

The flow velocity measurement control section 112 controls measurement of the flow velocity in the ascending aorta or the ventricular outflow tract. More specifically, the flow velocity measurement control section 112 controls transmission and reception of ultrasonic waves for implementing flow velocity measurement using the continuous-wave Doppler method, and performs a filtering process, an A/D conversion process, a frequency analysis process, and the like on the ultrasonic echo signal to calculate the flow velocity (V). The flow velocity measurement control section 112 may appropriately control ultrasonic beam forming, ultrasonic beam scanning, and the like. The flow velocity measurement module 102 illustrated in FIG. 1 corresponds to the flow velocity measurement control section 112.

The flow velocity peak value detection section 120 detects the (blood) flow velocity peak value (Vpeak (e.g., the value at the position indicated by the white circle in the waveform illustrated in FIG. 3)) within one cardiac cycle from flow velocity data 30 (see FIG. 3) measured by the continuous-wave Doppler method and output from the flow velocity measurement control section 112, and sequentially outputs the peak value to the cardiac output calculation section 130. The flow velocity peak value may be detected using a known technique that detects the peak value from time-series data. The flow velocity peak value detection section 120 may be implemented by an electronic circuit (e.g., LSI or ASIC), or may be implemented by a calculation process performed by a CPU or the like.

The arterial diameter measurement control section 114 controls measurement of the diameter of the artery (carotid artery in the first embodiment) using ultrasonic waves, and performs a filtering process, an A/D conversion process, a frequency analysis process, and the like on the ultrasonic echo signal to calculate the diameter of the artery (DOA). The arterial diameter measurement control section 114 may appropriately control ultrasonic beam forming, ultrasonic beam scanning, and the like. The arterial diameter measurement module 104 illustrated in FIG. 1 corresponds to the arterial diameter measurement control section 114. The arterial diameter measurement control section 114 may be implemented by an electronic circuit (e.g., LSI or ASIC), or may be implemented by a calculation process performed by a CPU or the like.

The ejection time/mean arterial pressure calculation section 122 calculates the ejection time (ET) and the mean arterial pressure (MAP) from the diameter of the artery (DOA) that is output from the arterial diameter measurement control section 114 in time series, outputs data about the ejection time (ET) to the cardiac output calculation section 130, and outputs data about the mean arterial pressure (MAP) to the cross-sectional area estimation section 124. The ejection time/mean arterial pressure calculation section 122 may be implemented by an electronic circuit (e.g., LSI or ASIC), or may be implemented by a calculation process performed by a CPU or the like.

As illustrated in FIG. 4, the ejection time/mean arterial pressure calculation section 122 detects the rising edge (Dus) and the dicrotic notch (Ddn) of a carotid artery diameter waveform 40, and calculates the ejection time (ET) from the time interval between the rising edge (Dus) and the dicrotic notch (Ddn), for example. The ejection time/mean arterial pressure calculation section 122 integrates and averages the arterial pressure calculated based on a known nonlinear function 46 over the calculated ejection time (ET) to calculate the mean arterial pressure (MAP). The ejection time/mean arterial pressure calculation section 122 outputs the calculated ejection time (ET) to the cardiac output calculation section 130, and outputs the mean arterial pressure (MAP) to the cross-sectional area estimation section 124. The rising edge (Dus) and the dicrotic notch (Ddn) of the carotid artery diameter waveform 40 may be detected by appropriately utilizing a known technique. For example, a technique that detects the rising edge and the dicrotic notch of an electrocardiogram may be used.

Since the flow velocity data 30 (see FIG. 3) obtained by measuring the blood flow velocity in the ascending aorta or the ventricular outflow tract using the ultrasonic Doppler method contains noise (e.g., a flow velocity signal in the diastolic phase or a clutter sound due to opening and closing of the valve), the waveform is vague at or around the ejection time start timing and the ejection time end timing, and it is considered that it is difficult to detect the ejection time (ET) from the flow velocity data 30. In contrast, since the waveform obtained from the diameter of the artery (carotid artery) (DOA) contains only a small amount of noise, the rising edge (Dus) and the dicrotic notch (Ddn) of the waveform are clear, and the ejection time (ET) can be automatically detected with high accuracy.

The cross-sectional area estimation section 124 estimates the cross-sectional area (CSA), and outputs the estimated cross-sectional area (CSA) of the aorta to the cardiac output calculation section 130. The cross-sectional area estimation section 124 may be implemented by an electronic circuit (e.g., LSI or ASIC), or may be implemented by a calculation process performed by a CPU or the like.

In the first embodiment, the cross-sectional area (CSA) is calculated using the arc tangent function arctan represented by the following equation (A) (see FIG. 5) (Journal of Applied Physiology, May 1, 1993, Vol. 74, No. 5, p. 2566).

$$CSA=CSAmax\cdot[0.5+(1/\pi)\cdot \arctan\{(MAP-P0)/P1\}] \qquad (A)$$

Note that the parameters CSAmax, P0, and P1 in the equation (A) are estimated from the age and the sex of the subject 3. A CSA parameter TBL (table) 156 that links these parameters corresponding to each age/sex combination is stored in the storage section 150, and the parameters CSAmax, P0, and P1 are referred to from the table data based on the age and the sex of the subject 3 that have been input using the keyboard 22.

Again referring to FIG. 2, the cardiac output calculation section 130 includes a stroke volume calculation section 132. The stroke volume calculation section 132 calculates the stroke volume (SV) from the flow velocity peak value (Vpeak) within one cardiac cycle, the ejection time (ET), and the cross-sectional area (CSA). The cardiac output calculation section 130 calculates the cardiac output (CO) by calculating a product of the stroke volume (SV) and the heart rate (HR).

Specifically, the cardiac output (CO) is calculated as a product of the stroke volume (SV) and the heart rate (HR) (see the following equation (B)) (see FIG. 6).

$$CO=HR\times SV \qquad (B)$$

The stroke volume (SV) can be calculated as a product of the cross-sectional area (CSA) and the velocity time integral (VTI) of the flow velocity in the systolic phase (see the following equation (C)).

$$SV=CSA\times VTI \quad (C)$$

The velocity time integral (VTI) is considered to be the area of one triangle indicated by the blood flow velocity, and is calculated by the following equation (D) using the flow velocity peak value (Vpeak) per beat as the height of the triangle, and using the ejection time (ET) as the length of the base of the triangle (Donnerstein et al., "Simplified method for estimation of Doppler cardiac output in the great arteries", The American Journal of Cardiology, July 1988, 62 (1), pp. 155-156).

$$VTI=Vpeak\times ET/2 \quad (D)$$

Therefore, the cardiac output calculation section 130 can calculate the cardiac output (CO) using the following equation (E).

$$CO=HR\times(Vpeak\times ET/2)\times CSA \quad (E)$$

Again referring to FIG. 2, the display control section 140 is implemented by hardware such as a graphics board provided with a GPU and a VRAM, or an electronic circuit (e.g., ASIC), or implemented by a calculation process performed by a CPU or the like, and performs a display control process that displays the cardiac output (CO) on the video monitor 20 (i.e., updates the cardiac output (CO) displayed on the video monitor 20) each time the cardiac output (CO) has been calculated. More specifically, the display control section 140 generates image data for displaying the cardiac output (CO) as a numerical value, or displaying the cardiac output (CO) in time-series graphical representation, generates an image signal for displaying the image data on the video monitor 20, and outputs the image signal to the video monitor 20. Note that the display control section 140 may also control display of additional information such as the electrocardiographic complex, the heart rate (HR), and the blood flow velocity waveform.

The alarm control section 142 is implemented by hardware (e.g., electronic circuit) or a calculation process performed by a CPU or the like, and displays a given alarm on the video monitor 20 when the calculated cardiac output (CO) has satisfied a given alarm condition. The alarm condition may be appropriately set using the threshold value or the change rate of the cardiac output (CO), for example. The alarm condition may be a fixed value, or may be an arbitrary value that is set using the keyboard 22 before starting the monitoring process.

The storage section 150 is implemented by an information storage medium such as an IC memory or a hard disk. The storage section 150 stores a system program 152, a monitoring program 154, and the CSA parameter TBL 156. The system program 152 is a basic program that causes the processing device 100 to function as a computer. Each functional section of the processing device 100 can be implemented by a calculation process by causing the processing device 100 to execute the monitoring program 154 in a state in which the system program 152 is executed.

Note that the storage section 150 functions as a data storage area common to each functional section, and can appropriately store a flag that is necessary for the calculation process performed by the processing device 100, a timing counter value, measured data, a calculated value and an estimated value (e.g., heart rate (HR), flow velocity peak value (Vpeak), system time that indicates the start timing of the ejection time (ET), system time that indicates the end timing of the ejection time (ET), mean arterial pressure (MAP), and cross-sectional area (CSA)), cardiac output calculation-related data 158 that includes various cardiac output calculation-related parameter values (e.g., the sex and the age of the subject), and the like.

FIG. 7 is a flowchart illustrating the flow of the process performed by the cardiac output monitoring system 5. Note that the operator attaches the electrodes 6, the transthoracic ultrasonic probe 10, and the arterial diameter measurement ultrasonic probe 12 to the subject 3, and connects the electrodes 6, the transthoracic ultrasonic probe 10, and the arterial diameter measurement ultrasonic probe 12 to the corresponding modules before starting the monitoring process.

The cardiac output monitoring system 5 performs a process that prompts the operator to input the sex and the age of the subject (step S6). For example, the cardiac output monitoring system 5 displays a given input screen on the video monitor 20, and prompts the operator to input the sex and the age of the subject 3 using the keyboard 22. The data about the sex and the age of the subject 3 input by the operator is stored in the storage section 150 as the cardiac output calculation-related data 158. The cross-sectional area estimation section 124 can refer to the data about the sex and the age of the subject 3.

The cardiac output monitoring system 5 then continuously measures the heart rate (HR) using the electrocardiography module 8, continuously measures the flow velocity using the flow velocity measurement module 102, and continuously measures the diameter of the artery using the arterial diameter measurement module 104 (step S10). In this case, the measured data may be stored in the storage section 150 in time series.

The cardiac output monitoring system 5 then calculates the heart rate (HR) based on the measured data (step S12), detects the flow velocity peak value (Vpeak) (step S14), calculates the ejection time (ET) and the mean arterial pressure (MAP) (step S16), and estimates the cross-sectional area (CSA) (step S18). These values may be linked to the measurement time, and stored in the storage section 150 in time series as the cardiac output calculation-related data 158. The cardiac output monitoring system 5 displays appropriate information (parameter value) (e.g., measured data and calculated value) on the video monitor 20 as a numerical value or in the form of a time-series waveform (step S20). The monitoring process starts in this manner.

When the ejection time (ET) has been updated after the monitoring process has started (YES in step S30), the cardiac output monitoring system 5 calculates the stroke volume (SV) (step S32), and multiplies the stroke volume (SV) by the latest heart rate (HR) to calculate the cardiac output (CO) (step S34). These values may be linked to the measurement time, and stored in the storage section 150 in time series. When a new cardiac output (CO) has been calculated, the cardiac output monitoring system 5 displays the new cardiac output (CO) on the video monitor 20 (step S36).

When the cardiac output (CO) has satisfied a given alarm condition (YES in step S40), the cardiac output monitoring system 5 performs an alarm control process, and displays an alarm on the video monitor 20 (step S42).

The cardiac output monitoring system 5 repeats the steps S30 to S42 in a given measurement cycle during a period in which the monitoring process is performed.

According to the first embodiment, the cardiac output monitoring system 5 can noninvasively and continuously monitor the cardiac output with high accuracy.

In the first embodiment, the parameter CSAmax used to calculate the cross-sectional area (CSA) is referred to from the CSA parameter TBL 156. Note that a value "CSAmax" measured before starting the monitoring process may be input (set) together with the age and the sex of the subject.

Specifically, the cross-sectional area (CSA) and the blood pressure may be measured by a known measurement method before starting the monitoring process. The measured cross-sectional area (CSA) may be substituted into the left side of the equation (A), the measured blood pressure may be substituted for the mean arterial pressure (MAP) on the right side of the equation (A), and the parameters P1 and P0 known from the age and the sex of the subject 3 may be substituted into the right side of the equation (A) to calculate the value "CSAmax". The value "CSAmax" thus calculated may be input in the step S6 in the same manner as the age and the sex of the subject. The input value "CSAmax" may be stored in the storage section 150 as the cardiac output calculation-related data 158. The cross-sectional area estimation section 124 may refer to the CSA parameter TBL 156 for the values P1 and P0 corresponding to the age and the sex of the subject 3, refer to the cardiac output calculation-related data 158 for the value "CSAmax", and calculate the cross-sectional area (CSA) using the equation (A).

## Second embodiment

A second embodiment to which the invention is applied is described below. The configuration according to the second embodiment is basically the same as the configuration according to the first embodiment, but differs from the configuration according to the first embodiment in that the cross-sectional area (CSA) is calculated directly from the measured diameter of the artery. Note that the following description mainly focuses on the differences from the first embodiment. The same elements as those described above in connection with the first embodiment are indicated by identical reference signs, and detailed description thereof is omitted.

FIG. 8 is a functional block diagram illustrating a functional configuration example of a cardiac output monitoring system 5B according to the second embodiment. A processing device 100B included in the cardiac output monitoring system 5B includes an ejection time calculation section 123 instead of the ejection time/mean arterial pressure calculation section 122 described above in connection with the first embodiment.

The ejection time calculation section 123 calculates the ejection time (ET) from the diameter of the artery (carotid artery) (DOA) that is output from the arterial diameter measurement control section 114 in time series, and outputs data about the ejection time (ET) to the cardiac output calculation section 130. The ejection time calculation section 123 outputs time-series arterial diameter data calculated during a period from the rising edge (Dus) to the dicrotic notch (Ddn) of the carotid artery diameter waveform 40 to a cross-sectional area estimation section 124B.

As illustrated in FIG. 9, the cross-sectional area estimation section 124B calculates the cross-sectional area (CSA) during a period between the rising edge (Dus) and the dicrotic notch (Ddn) of the carotid artery diameter waveform 40 using the following equation (G) based on a nonlinear function 48, integrates and averages the cross-sectional area (CSA) over the ejection time (ET), and outputs the resulting value to the cardiac output calculation section 130 as the cross-sectional area (CSA) during the ejection time (ET).

$$CSA = A \times DOA^2 + B \times DOA + C \qquad (G)$$

Note that A, B, and C are given constants. The constants A, B, and C may be set in advance in a program, or may be stored in the storage section 150 in advance so that the constants A, B, and C can be referred to. The experimental results obtained by the applicants suggest that the constant A may be 4.8, the constant B may be -41.3, and the constant C may be 89.7, for example. Note that the combination of these coefficients may be determined by experiments. An average integral value over the ejection time (ET) may be used as the cross-sectional area (CSA).

The flow of the process performed by the cardiac output monitoring system 5B is basically the same as that described above in connection with the first embodiment. Note that the sex and the age of the subject 3 need not necessarily be input. The second embodiment can thus achieve the same advantageous effects as those achieved by the first embodiment. The elements may be added, changed, omitted, or replaced in the same manner as in the first embodiment.

Third embodiment

A third embodiment to which the invention is applied is described below. The configuration according to the third embodiment is basically the same as the configuration according to the first embodiment, but differs from the configuration according to the first embodiment in that the ejection time (ET) and the mean arterial pressure (MAP) are calculated from the arterial pressure of a third artery that can be detected from the body surface, and the cross-sectional area (CSA) is estimated from the arterial pressure of the third artery. Note that the following description mainly focuses on the difference from the first embodiment. The same elements as those described above in connection with the first embodiment are indicated by identical reference signs, and detailed description thereof is omitted.

FIG. 10 is a view illustrating a system configuration example of a cardiac output monitoring system 5C according to the third embodiment. The cardiac output monitoring system 5C includes (1) an electrocardiography module 8 that continuously generates and outputs an electrocardiogram waveform (ECG waveform) that is obtained through electrodes 6 attached to the surface of the chest of a subject 3, and indicates a temporal change in electrical activity of the heart, (2) a transthoracic ultrasonic probe 10 that is a first sensor section that measures the blood flow velocity in a first artery or the ventricular outflow tract connected to the first artery from the body surface of the subject 3, (3) a peripheral arterial pressure measurement probe 14 that is a third sensor section that measures the arterial pressure of a third artery that can be detected from the body surface of the subject 3, (4) a processing device 100C that continuously calculates and outputs the cardiac output based on the data measured by the electrocardiography module 8, the transthoracic ultrasonic probe 10, and the peripheral arterial pressure measurement probe 14, (5) a video monitor 20, and (6) a keyboard 22.

The processing device 100C includes a peripheral arterial pressure measurement module 106 that measures the peripheral arterial pressure using the peripheral arterial pressure measurement probe 14 instead of the arterial diameter measurement module 104 described above in connection with the first embodiment.

The peripheral arterial pressure measurement probe 14 is a sensor that is implemented by known strain sensor technology or the like, and is inserted into the radial artery of the subject 3 to measure the blood pressure in the radial artery.

FIG. 11 is a functional block diagram illustrating a functional configuration example according to the third embodiment. The processing device 100C according to the third embodiment includes an arterial pressure measurement control section 116 instead of the arterial diameter measurement control section 114 described above in connection with the first embodiment.

The arterial pressure measurement control section 116 derives the arterial pressure (P) from an electrical signal output from a strain sensor. Specifically, the arterial pressure measurement control section 116 functions as a blood pressure-deriving section. The peripheral arterial pressure measurement module 106 illustrated in FIG. 10 corresponds to the arterial pressure measurement control section 116.

An ejection time/mean arterial pressure calculation section 122C calculates the ejection time (ET) and the mean arterial pressure (MAP) from peripheral artery blood pressure data that is output from the arterial pressure measurement control section 116 in time series, outputs data about the ejection time (ET) to the cardiac output calculation section 130, and outputs data about the mean arterial pressure (MAP) to the cross-sectional area estimation section 124.

As illustrated in FIG. 12, the ejection time/mean arterial pressure calculation section 122C detects the rising edge (Pus) and the dicrotic notch (Pdn) indicated by peripheral artery blood pressure data 42 that is output from the arterial pressure measurement control section 116 in time series, and measures the time elapsed from the rising edge (Pus) to the dicrotic notch (Pdn) to determine the ejection time (ET), for example.

Since the flow velocity data 30 (see FIG. 3) obtained by measuring the blood flow velocity in the aorta using the ultrasonic Doppler method contains noise (e.g., a flow velocity signal in the diastolic phase or a clutter sound due to opening and closing of the valve), the waveform is vague at or around the ejection time start timing and the ejection time end timing, and it is considered that it is difficult to automatically detect the ejection time (ET) from the flow velocity data 30. In contrast, since the peripheral arterial pressure waveform contains a relatively small amount of noise, the rising edge (Pus) and the dicrotic notch (Pdn) of the peripheral arterial pressure waveform can be automatically detected. Therefore, the ejection time (ET) can be accurately detected as compared with the case of detecting the ejection time (ET) from the flow velocity data 30 about the ventricular outflow tract. The mean arterial pressure (MAP) is calculated as the average arterial pressure over the ejection time (ET).

The flow of the process performed by the cardiac output monitoring system 5C is basically the same as that described above in connection with the first embodiment. The third embodiment can thus achieve the same advantageous effects as those achieved by the first embodiment. The elements may be added, changed, omitted, or replaced in the same manner as in the first embodiment.

Fourth embodiment

A fourth embodiment to which the invention is applied is described below. The configuration according to the fourth embodiment is basically the same as the configuration according to the second embodiment, but differs from the configuration according to the second embodiment in that the cross-sectional area (CSA) is estimated based on the diameter of the artery, and the ejection time (ET) is calculated from the arterial pressure of the third artery (see the third embodiment). Note that the following description mainly focuses on the differences from the second embodiment. The same elements as those described above in connection with the second embodiment are indicated by identical reference signs, and detailed description thereof is omitted.

FIG. 13 is a view illustrating a system configuration example of a cardiac output monitoring system 5D according to the fourth embodiment. The cardiac output monitoring system 5D includes (1) an electrocardiography module 8 that continuously generates and outputs an electrocardiogram waveform (ECG waveform) that is obtained through electrodes 6 attached to the surface of the chest of a subject 3, and indicates a temporal change in electrical activity of the heart, (2) a transthoracic ultrasonic probe 10 that is a first sensor section that measures the blood flow velocity in a first artery or the ventricular outflow tract connected to the first artery from the body surface of the subject 3, (3) an arterial diameter measurement ultrasonic probe 12 that is a second sensor section that measures the diameter of a second artery from the body surface of the subject 3, (4) a peripheral arterial pressure measurement probe 14 that is a third sensor section that measures the arterial pressure of a third artery, (5) a processing device 100D that continuously calculates and outputs the cardiac output based on the data measured by the electrocardiography module 8, the transthoracic ultrasonic probe 10, the arterial diameter measurement ultrasonic probe 12, and the peripheral arterial pressure measurement probe 14, (6) a video monitor 20, and (7) a keyboard 22.

The processing device 100D includes the flow velocity measurement module 102 described above in connection with the first embodiment, the arterial diameter measurement module 104 described above in connection with the second embodiment, and the peripheral arterial pressure measurement module 106 described above in connection with the third embodiment.

FIG. 14 is a functional block diagram illustrating a functional configuration example according to the fourth embodiment. The processing device 100D according to the fourth embodiment includes a heart rate calculation section 110, a flow velocity measurement control section 112, an arterial diameter measurement control section 114, and an arterial pressure measurement control section 116. The arterial pressure measurement control section 116 outputs the arterial pressure (P) to an ejection time calculation section 123D.

The ejection time calculation section 123D calculates the ejection time (ET) from a temporal change in the detection signal output from the peripheral arterial pressure measurement probe 14, and outputs the ejection time (ET) to the cardiac output calculation section 130 in the same manner as the ejection time/mean arterial pressure calculation section 122C described above in connection with the third embodiment. The ejection time calculation section 123D outputs the arterial pressure at the rising edge (Pus) of the carotid artery diameter waveform 40 and the arterial pressure at the dicrotic notch (Pdn) of the carotid artery diameter waveform 40 calculated when calculating the ejection time (ET), to the cross-sectional area estimation section 124B in the same manner as the ejection time/mean arterial pressure calculation section 122C described above in connection with the third embodiment.

The cross-sectional area estimation section 124B calculates the cross-sectional area (CSA) during a period between the rising edge (Dus) and the dicrotic notch (Ddn) of the carotid artery diameter waveform 40 that respectively correspond to the arterial pressure at the rising edge (Pus) and the arterial pressure at the dicrotic notch (Pdn) based on the nonlinear function 48 (see FIG. 9), integrates and averages the cross-sectional area (CSA) over the ejection time (ET), and outputs the resulting value to the cardiac output calculation section 130 as the cross-sectional area (CSA) during the ejection time (ET).

The flow of the process performed by the cardiac output monitoring system 5D is basically the same as that described above in connection with the second embodiment. That is, the flow of the process performed by the cardiac output monitoring system 5D is basically the same as that described above in connection with the first embodiment.

Therefore, the fourth embodiment can achieve the same advantageous effects as those achieved by the first embodiment. The elements may be added, changed, omitted, or replaced in the same manner as in the first embodiment.

The cardiac output measurement test results

FIGS. 15 and 16 are graphs showing the cardiac output measurement test results obtained using the cardiac output monitoring system 5C according to the third embodiment. The cardiac output measurement test was performed in a state in which an aortic flow velocity probe was implanted directly into the ascending aorta of each of eight dogs, the electrocardiographic electrodes 6, the transthoracic ultrasonic probe 10, and the peripheral artery pressure measurement probe 14 were attached to each dog, and the hemodynamics were changed to a large extent using a cardiotonic drug or the like.

FIG. 15 is a graph showing the goodness of tracking of the cardiac output of one representative dog among the eight dogs. It was confirmed that the value (COest) measured by the cardiac output monitoring system 5C according to the third embodiment sufficiently followed a change in the value (COref) measured using the aortic flow velocity probe (i.e., a high-fidelity response was obtained by the cardiac output monitoring system 5C according to the third embodiment).

FIG. 16 is a graph showing the absolute value of the value (COest) of each dog measured by the cardiac output monitoring system 5C according to the third embodiment and the absolute value of the value (COref) of each dog measured using the aortic flow velocity probe, and a relative change rate from the initial value. It was confirmed that the absolute values sufficiently coincide with each other.

It was thus demonstrated that the cardiac output monitoring system can noninvasively and continuously monitor the cardiac output with high accuracy.

Modifications

The embodiments to which the invention is applied have been described above. Note that the invention is not limited thereto. Various modifications may be appropriately made, such as adding other elements, omitting some of the elements, or replacing some of the elements.

As illustrated in FIG. 17, the transthoracic ultrasonic probe 10 described in connection with the above embodiments may be a thin and flat ultrasonic probe for which the focus of the ultrasonic beam can be changed in three-dimensional directions, and the flow velocity measurement module 102 may include an ultrasonic beam control section 184.

More specifically, the flow velocity measurement module 102 may be configured so that a rate pulse generated by a rate pulse generation section 170 is delayed by a transmission delay circuit 172, and output to a drive control circuit 174 that generates a drive signal for each ultrasonic transducer in the same manner as in the case of measuring the flow velocity using a known continuous-wave Doppler method. The ultrasonic beam can be formed to achieve an in-focus state, or the scan direction or range can be controlled by controlling the amount of delay using the transmission delay circuit 172, or selectively driving the ultrasonic transducer using the drive control circuit 174. The ultrasonic echo is amplified by a preamplifier 176, and stored in an echo storage section 178. The ultrasonic echo stored in the echo storage section 178 is subjected to a delay process by a reception delay summation section 180 in order to adjust the directivity to the echo from the focus position, and transmitted to a flow velocity data calculation section 182. The flow velocity data calculation section 182 performs a Doppler shift frequency analysis process to calculate and output flow velocity data.

The ultrasonic beam control section 184 is implemented by a DSP, an ASIC, or a calculation process performed by a CPU. The ultrasonic beam control section 184 controls either or both of ultrasonic beam focusing and ultrasonic beam scanning performed by the transthoracic ultrasonic probe 10 so that the temporal change waveform of the flow velocity satisfies a given clearness condition and/or the flow velocity peak value becomes a maximum. Specifically, the ultrasonic beam control section 184 controls either or both of ultrasonic beam focusing and ultrasonic beam scanning so that the flow velocity can be measured more accurately.

The ultrasonic beam control section 184 outputs a delay pattern signal for implementing the desired control to the transmission delay circuit 172 and the reception delay summation section 180, outputs appropriate rate information to the rate pulse generation section 170, and outputs a selection signal that selects the drive target ultrasonic transducer to the drive control circuit 174. The transmission delay circuit 172 delays the transmission signal according to the delay pattern signal. The reception delay summation section 180 applies a delay time to the echo signal based on the delay pattern signal. The drive control circuit 174 drives the selected ultrasonic transducer. The ultrasonic beam control section 184 may utilize a technique that controls either or both of ultrasonic beam focusing and ultrasonic beam scanning to trace the maximum flow velocity in the measurement range taking account of the intended use of the flow velocity measured by the flow velocity measurement module 102.

Therefore, when the flow velocity measurement module 102 includes the ultrasonic beam control section 184, the measurement accuracy is always maintained even if the relative position or the relative posture of the transthoracic ultrasonic probe 10 is changed during continuous cardiac output measurement due to a change in posture of the subject or the like.

As illustrated in FIG. 18, a second ultrasonic beam control section 186 may be provided in the arterial diameter measurement module 104 described in connection with the above embodiments in order to achieve the above effect.

The second ultrasonic beam control section 186 is implemented by a DSP, an ASIC, or a calculation process performed by a CPU. The second ultrasonic beam control section 186 controls either or both of ultrasonic beam focusing and ultrasonic beam scanning performed by the arterial diameter measurement ultrasonic probe 12 so that the major axis length and the minor axis length satisfy a given equality condition when the cross section of a given artery that intersects the direction in which the given artery extends is elliptical, and/or the cross section satisfies a given circularity condition. In other words, the second ultrasonic beam control section 186 adjusts and controls ultrasonic beam forming or ultrasonic beam scanning to scan the cross section that is almost circular and intersects the direction in which the artery extends so that the diameter of the artery can be accurately measured.

The second ultrasonic beam control section 186 may utilize a technique that calculates a cross section (orthogonal cross section) orthogonal to the direction in which the blood vessel extends (blood vessel major axis), and a technique that controls beam forming or beam scanning so that the shape of the blood vessel can be measured at the desired cross section.

For example, the arterial diameter measurement ultrasonic probe 12 may be provided with a two-dimensional array in which a plurality of ultrasonic transducers are disposed in a matrix, and the operator may attach the arterial diameter measurement ultrasonic probe 12 at a position over the carotid artery. The second ultrasonic beam control section 186 scans the three-dimensional area of the carotid artery to acquire volume data, and calculates the blood vessel centerline and a cross section orthogonal to the centerline. The second ultrasonic beam control section 186 may utilize a known technique that controls beam forming or beam scanning so that the shape of the blood vessel can be measured at the cross section orthogonal to the centerline.

Alternatively, the arterial diameter measurement ultrasonic probe 12 may be provided with a plurality of parallel line arrays in which a plurality of ultrasonic transducers are arranged linearly, and the operator may attach the arterial diameter measurement ultrasonic probe 12 so that the line arrays are orthogonal to the direction in which the carotid artery extends. The measurement may be performed on a line array basis, and the diameter of the blood vessel is estimated from the cross-sectional measurement results based on the tilt angle of each cross section with the blood vessel centerline.

As illustrated in FIG. 19, the control process performed by the ultrasonic beam control section 184 and the control process performed by the second ultrasonic beam control section 186 may be provided between the step S20 and the step S30, for example. In a step S22, whether or not it is necessary to readjust the measurement parameter (readjustment necessary state) is determined.

The term "readjustment necessary state" used herein in connection with the ultrasonic beam control section 184 refers to a state in which the temporal change waveform of the flow velocity does not satisfy a given clearness condition and/or the flow velocity peak value does not become a maximum. The term "readjustment necessary state" used herein in connection with the second ultrasonic beam control section 186 refers to a state in which the major axis length and the minor axis length do not satisfy a given equality condition when the cross section of the artery that intersects the direction in which the artery extends is elliptical, and/or the cross section does not satisfy a given circularity condition. Whether or not the temporal change waveform of the flow velocity satisfies the clearness condition may be determined by utilizing an image processing technique that determines whether or not a triangular shape is observed in the temporal change waveform of the flow velocity.

When it has been determined that it is necessary to readjust the measurement parameter (YES in step S22), an ultrasonic beam optimization control process is performed (step S24). Specifically, the ultrasonic beam control section 184 and the second ultrasonic beam control section 186 perform the control process corresponding to the measured data that requires a readjustment.

Note that the peripheral arterial pressure measurement probe 14 may be implemented by an ultrasonic probe. In this case, the peripheral arterial pressure measurement probe 14 may be a thin and flat pad-type sensor that includes a thin-film piezoelectric diaphragm-type ultrasonic transducer, and can be attached to the subject 3 at a position over the radial artery to measure the diameter of the radial artery, a functional section that corresponds to the second ultrasonic beam control section 186 may be provided in the peripheral arterial pressure measurement module 106, and the arterial pressure may be measured based on the measured diameter of the blood vessel using the relationship between the diameter of the blood vessel and the arterial pressure specified in advance.

The measurement of the peripheral arterial pressure described in connection with the above embodiments may not be performed using a strain sensor, and may be replaced with estimation from the diameter of the artery using ultrasonic waves, or measurement of the arterial pressure using infrared rays or the like.

Although the above embodiments have been described taking an example in which the heart rate (HR) is calculated from an electrocardiogram, the heart rate (HR) may be calculated from the time interval between the flow velocity peak values, a temporal change in diameter of the artery, or a temporal change in arterial pressure by appropriately utilizing a known technique. In this case, the elements required to measure an electrocardiogram can be omitted.

Although only some embodiments of the present invention have been described in detail above, those skilled in the art will readily appreciate that many modifications are possible in the embodiments without materially departing from the novel teachings and advantages of this invention. Accordingly, all such modifications are intended to be included within scope of this invention.

## Claims

**1.** A cardiac output monitoring system comprising:

a flow velocity peak value detection section that detects a flow velocity peak value, the flow velocity peak value being a peak value of a flow velocity in a first artery or a ventricular outflow tract that has been measured from a body surface using a first sensor section;
an ejection time calculation section that calculates an ejection time from a temporal change in diameter of an artery, the diameter of the artery being a diameter of a second artery that has been measured from a body surface using a second sensor section;
a cross-sectional area estimation section that estimates a cross-sectional area of the first artery from the diameter of the second artery using a relationship between the diameter of the second artery and the cross-sectional area of the first artery that has been specified in advance;
a stroke volume calculation section that calculates a stroke volume using the flow velocity peak value, the ejection time, and the cross-sectional area; and
a cardiac output calculation section that calculates a cardiac output using the stroke volume and a given heart rate.

**2.** A cardiac output monitoring system comprising:

a flow velocity peak value detection section that detects a flow velocity peak value, the flow velocity peak value being a peak value of a flow velocity in a first artery or a ventricular outflow tract that has been measured from a body surface using a first sensor section;
an ejection time calculation section that calculates an ejection time from a temporal change in arterial pressure, the arterial pressure being a pressure of a third artery using a third sensor section, the third artery being an artery that is palpable from a body surface;
a cross-sectional area estimation section that estimates a cross-sectional area of the first artery from the arterial pressure of the third artery using a relationship between the arterial pressure of the third artery and the cross-sectional area of the first artery that has been specified in advance;
a stroke volume calculation section that calculates a stroke volume using the flow velocity peak value, the ejection time, and the cross-sectional area; and
a cardiac output calculation section that calculates a cardiac output using the stroke volume and a given heart rate.

**3.** A cardiac output monitoring system comprising:

a flow velocity peak value detection section that detects a flow velocity peak value, the flow velocity peak value being a peak value of a flow velocity in a first artery or a ventricular outflow tract that has been measured from a body surface using a first sensor section;
a cross-sectional area estimation section that estimates a cross-sectional area of the first artery from a diameter of a second artery using a relationship between the diameter of the second artery and the cross-sectional area of the first artery that has been specified in advance, the diameter of the second artery being a diameter that has been measured from a body surface using a second sensor section;
an ejection time calculation section that calculates an ejection time from a temporal change in arterial pressure, the arterial pressure being a pressure of a third artery using a third sensor section, the third artery being an artery that is palpable from a body surface;
a stroke volume calculation section that calculates a stroke volume using the flow velocity peak value, the ejection time, and the cross-sectional area; and
a cardiac output calculation section that calculates a cardiac output using the stroke volume and a given heart rate.

**4.** The cardiac output monitoring system as defined in any one of claims 1 to 3,
the first sensor section being a sensor that transmits and receives an ultrasonic beam,
the cardiac output monitoring system further comprising a first ultrasonic beam control section that controls either or both of ultrasonic beam focusing and ultrasonic beam scanning performed by the first sensor section so that (a) a temporal change waveform of the flow velocity based on a detection signal of the first sensor section satisfies a given clearness condition or (b) the flow velocity peak value detected by the flow velocity peak value detection section becomes a maximum, or (c) the temporal change waveform of the flow velocity based on the detection signal of the first sensor section satisfies the given clearness condition and the flow velocity peak value detected by the flow velocity peak value detection section becomes a maximum.

**5.** The cardiac output monitoring system as defined in claim 1 or 3,
the second sensor section being a sensor that transmits and receives an ultrasonic beam,
the cardiac output monitoring system further comprising a second ultrasonic beam control section that controls either or both of ultrasonic beam focusing and ultrasonic beam scanning performed by the second sensor section so that (a) a major axis length and a minor axis length of a cross section of the second artery satisfy a given equality condition or (b) the cross section satisfies a given circularity condition, or (c) the major axis length and the minor axis length of the cross section of the second artery satisfy the given equality condition and the cross section satisfies the given circularity condition.

**6.** The cardiac output monitoring system as defined in any one of claims 1 to 5,
the first sensor section being a thin and flat ultrasonic probe that is attached to a surface of a chest wall of a subject, and configured so that a focus of an ultrasonic beam can be changed in three-dimensional directions.

**7.** The cardiac output monitoring system as defined in any one of claims 1 to 6, further comprising:

a display control section that updates and displays the cardiac output each time the cardiac output has been calculated by the cardiac output calculation section; and
an alarm control section that gives a given alarm when the cardiac output calculated by the cardiac output calculation section has satisfied a given alarm condition.

**8.** A cardiac output measurement method comprising:

detecting a flow velocity peak value, the flow velocity peak value being a peak value of a flow velocity in a first artery or a ventricular outflow tract that has been measured from a body surface using a first sensor section;
calculating an ejection time from a temporal change in diameter of an artery, the diameter of the artery being a diameter of a second artery that has been measured from a body surface using a second sensor section;
estimating a cross-sectional area of the first artery from the diameter of the second artery using a relationship between the diameter of the second artery and the cross-sectional area of the first artery that has been specified in advance;
calculating a stroke volume using the flow velocity peak value, the ejection time, and the cross-sectional area; and
calculating a cardiac output using the stroke volume and a given heart rate.

**9.** A cardiac output measurement method comprising:

detecting a flow velocity peak value, the flow velocity peak value being a peak value of a flow velocity in a first artery or a ventricular outflow tract that has been measured from a body surface using a first sensor section;
calculating an ejection time from a temporal change in arterial pressure, the arterial pressure being a pressure of a third artery using a third sensor section, the third artery being an artery that is palpable from a body surface;
estimating a cross-sectional area of the first artery from the arterial pressure of the third artery using a relationship between the arterial pressure of the third artery and the cross-sectional area of the first artery that has been specified in advance;
calculating a stroke volume using the flow velocity peak value, the ejection time, and the cross-sectional area; and
calculating a cardiac output using the stroke volume and a given heart rate.

**10.** A cardiac output measurement method comprising:

detecting a flow velocity peak value, the flow velocity peak value being a peak value of a flow velocity in a first artery or a ventricular outflow tract that has been measured from a body surface using a first sensor section;
estimating a cross-sectional area of the first artery from a diameter of a second artery using a relationship between the diameter of the second artery and the cross-sectional area of the first artery that has been specified in advance, the diameter of the second artery being a diameter that has been measured from a body surface using a second sensor section;
calculating an ejection time from a temporal change in arterial pressure, the arterial pressure being a pressure of a third artery using a third sensor section, the third artery being an artery that is palpable from a body surface;
calculating a stroke volume using the flow velocity peak value, the ejection time, and the cross-sectional area; and
calculating a cardiac output using the stroke volume and a given heart rate.

**11.** The cardiac output measurement method as defined in claim 8 or 10,
the estimation of the cross-sectional area including estimating the cross-sectional area using an equation (1),

$$\text{cross-sectional area}=A\times DOA^2+B\times DOA+C \qquad (1)$$

where, DOA is the diameter of the second artery indicated by a detection signal of the second sensor section, and A, B, and C are constants set in advance, and
the calculating of the stroke volume including calculating the stroke volume using an equation (2),

$$\text{stroke volume}=(Vpeak\times ET/2)\times CSA \qquad (2)$$

where, Vpeak is the detected flow velocity peak value, ET is the calculated ejection time, and CSA is the estimated cross-sectional area.

**12.** The cardiac output measurement method as defined in claim 9,
the estimation of the cross-sectional area including estimating the cross-sectional area using an equation (3),

$$\text{cross-sectional area}=E\times\{0.5+(1/\pi)\times\arctan[(AP-F)/G]\} \qquad (3)$$

where, arctan is an arc tangent function, AP is the arterial pressure of the third artery indicated by a detection signal of the third sensor section, and E, F, and G are constants set in advance, and
the calculation of the stroke volume including calculating the stroke volume using an equation (4),

$$\text{stroke volume}=(Vpeak\times ET/2)\times CSA \qquad (4)$$

where, Vpeak is the detected flow velocity peak value, ET is the calculated ejection time, and CSA is the estimated cross-sectional area.

FIG. 1

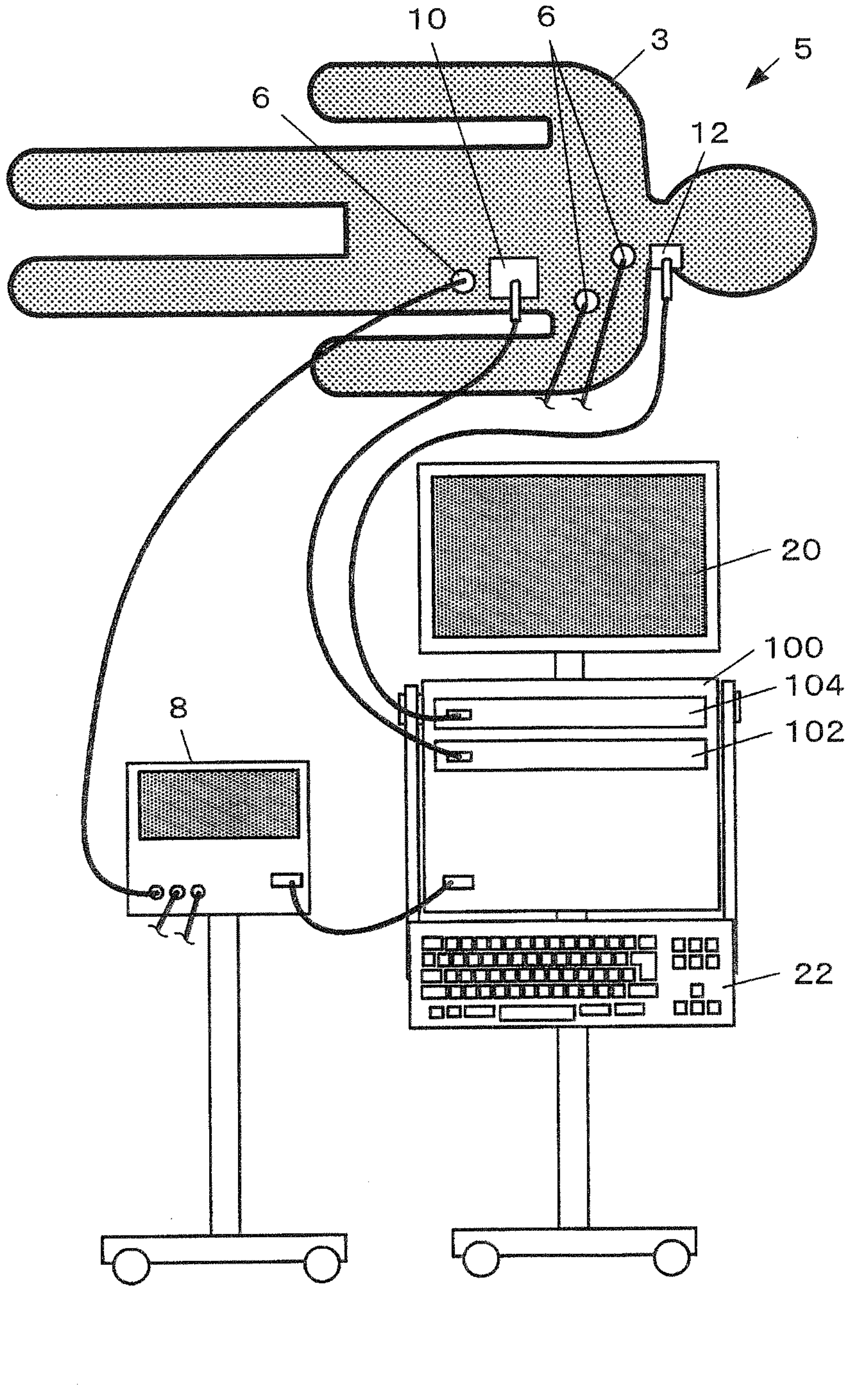

FIG. 2

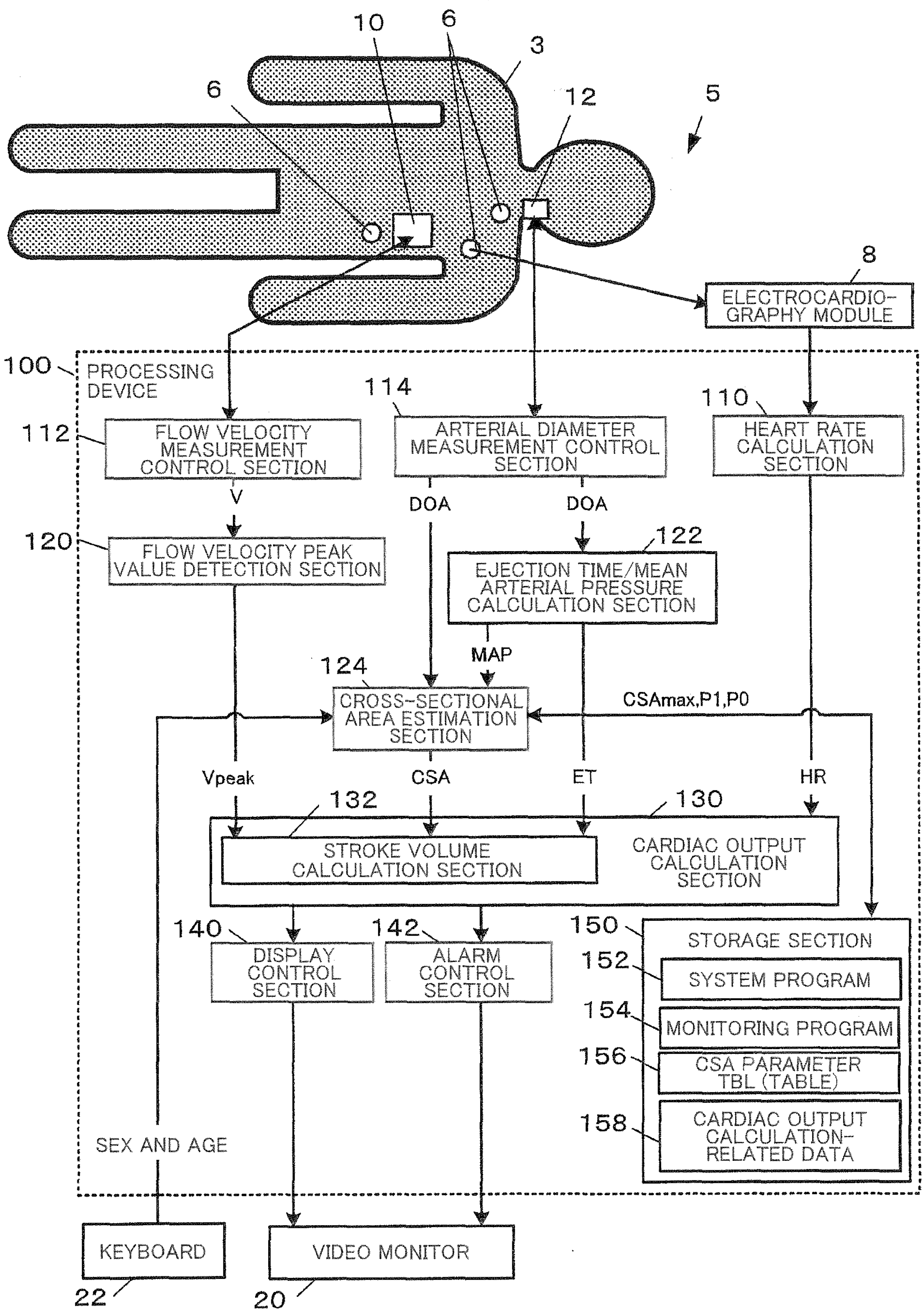
10
6
3
6
12
5
8
ELECTROCARDIO-GRAPHY MODULE
100
PROCESSING DEVICE
114
110
112
FLOW VELOCITY MEASUREMENT CONTROL SECTION
ARTERIAL DIAMETER MEASUREMENT CONTROL SECTION
HEART RATE CALCULATION SECTION
V
DOA
DOA
122
120
FLOW VELOCITY PEAK VALUE DETECTION SECTION
EJECTION TIME/MEAN ARTERIAL PRESSURE CALCULATION SECTION
MAP
124
CROSS-SECTIONAL AREA ESTIMATION SECTION
CSAmax,P1,P0
Vpeak
CSA
ET
HR
132
130
STROKE VOLUME CALCULATION SECTION
CARDIAC OUTPUT CALCULATION SECTION
140
142
150
DISPLAY CONTROL SECTION
ALARM CONTROL SECTION
STORAGE SECTION
152
SYSTEM PROGRAM
154
MONITORING PROGRAM
156
CSA PARAMETER TBL (TABLE)
158
CARDIAC OUTPUT CALCULATION-RELATED DATA
SEX AND AGE
KEYBOARD
VIDEO MONITOR
22
20

FIG. 3

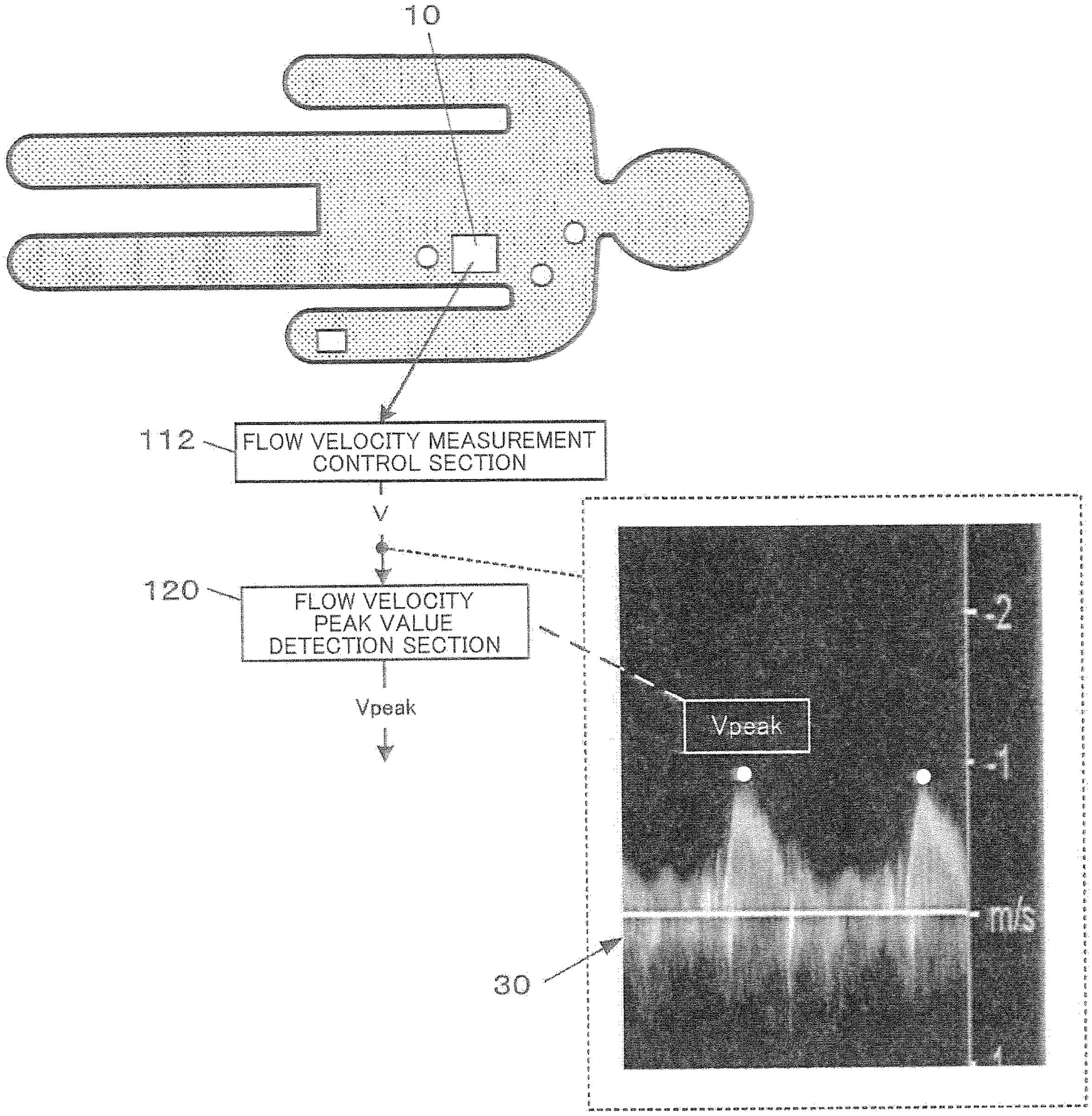
10
112
FLOW VELOCITY MEASUREMENT CONTROL SECTION
V
120
FLOW VELOCITY PEAK VALUE DETECTION SECTION
Vpeak
Vpeak
-2
-1
m/s
30

FIG. 4

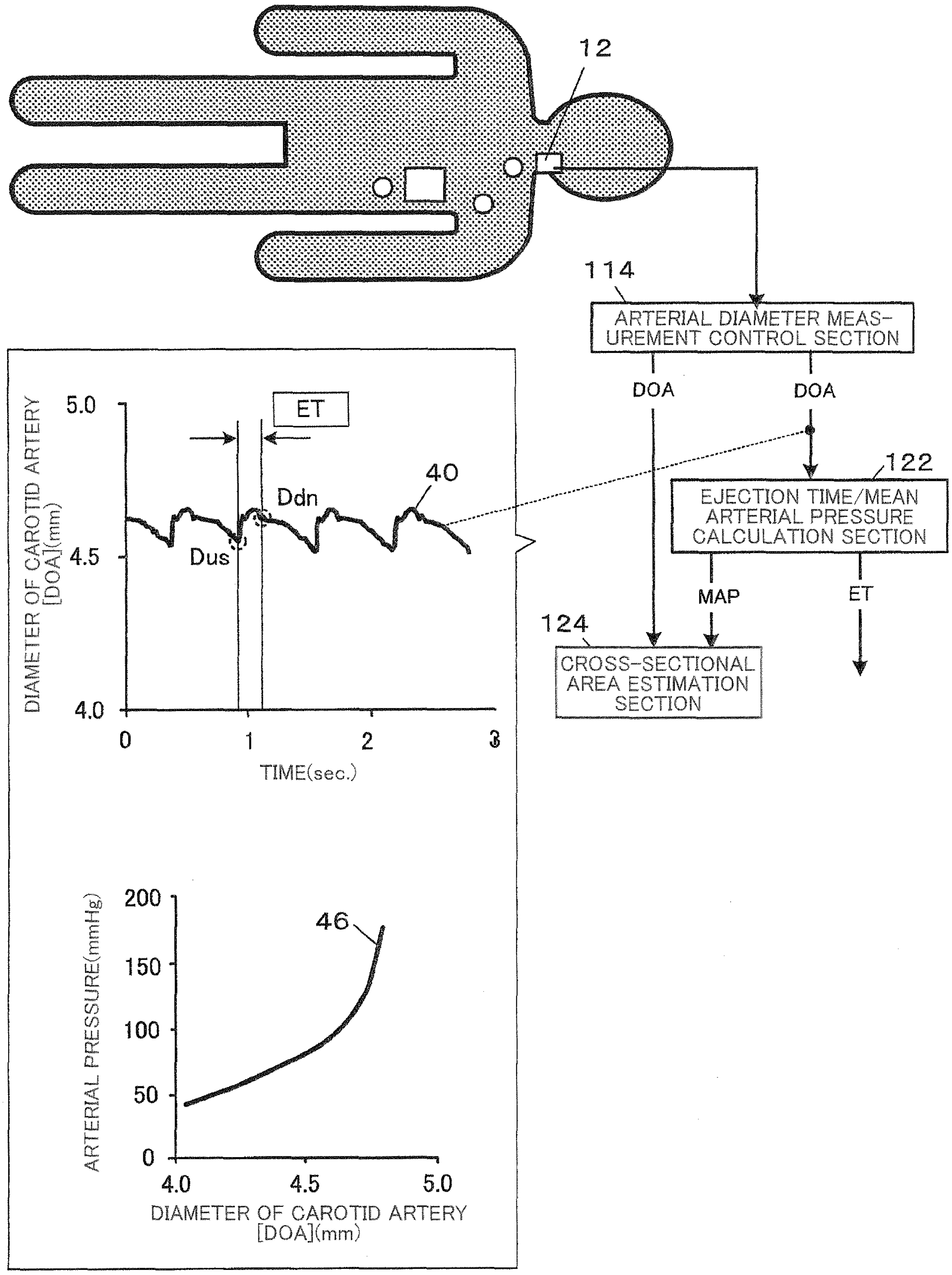
12
114
ARTERIAL DIAMETER MEASUREMENT CONTROL SECTION
DOA
DOA
122
EJECTION TIME/MEAN ARTERIAL PRESSURE CALCULATION SECTION
MAP
ET
124
CROSS-SECTIONAL AREA ESTIMATION SECTION
DIAMETER OF CAROTID ARTERY [DOA](mm)
5.0
4.5
4.0
ET
40
Ddn
Dus
0
1
2
3
TIME(sec.)
ARTERIAL PRESSURE(mmHg)
200
150
100
50
0
46
4.0
4.5
5.0
DIAMETER OF CAROTID ARTERY [DOA](mm)

FIG. 5

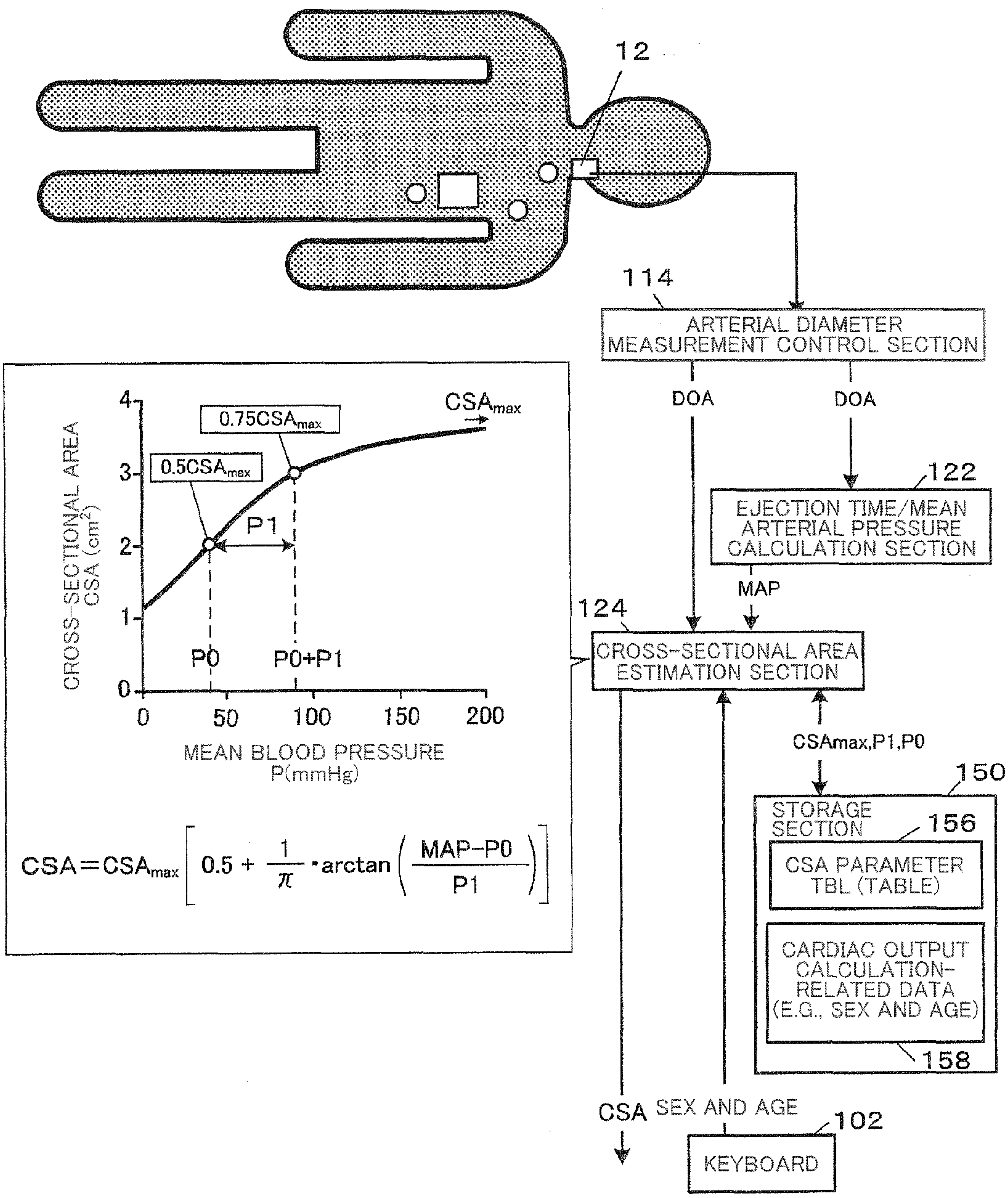
12
114
ARTERIAL DIAMETER MEASUREMENT CONTROL SECTION
DOA
DOA
122
EJECTION TIME/MEAN ARTERIAL PRESSURE CALCULATION SECTION
MAP
124
CROSS-SECTIONAL AREA ESTIMATION SECTION
CSAmax,P1,P0
150
STORAGE SECTION
156
CSA PARAMETER TBL (TABLE)
CARDIAC OUTPUT CALCULATION-RELATED DATA (E.G., SEX AND AGE)
158
CSA
SEX AND AGE
102
KEYBOARD
CROSS-SECTIONAL AREA CSA (cm2)
0.75CSAmax
0.5CSAmax
CSAmax
P1
P0
P0+P1
0
1
2
3
4
0
50
100
150
200
MEAN BLOOD PRESSURE P(mmHg)
CSA=CSAmax [0.5 + 1/π · arctan ((MAP−P0)/P1)]

FIG. 6

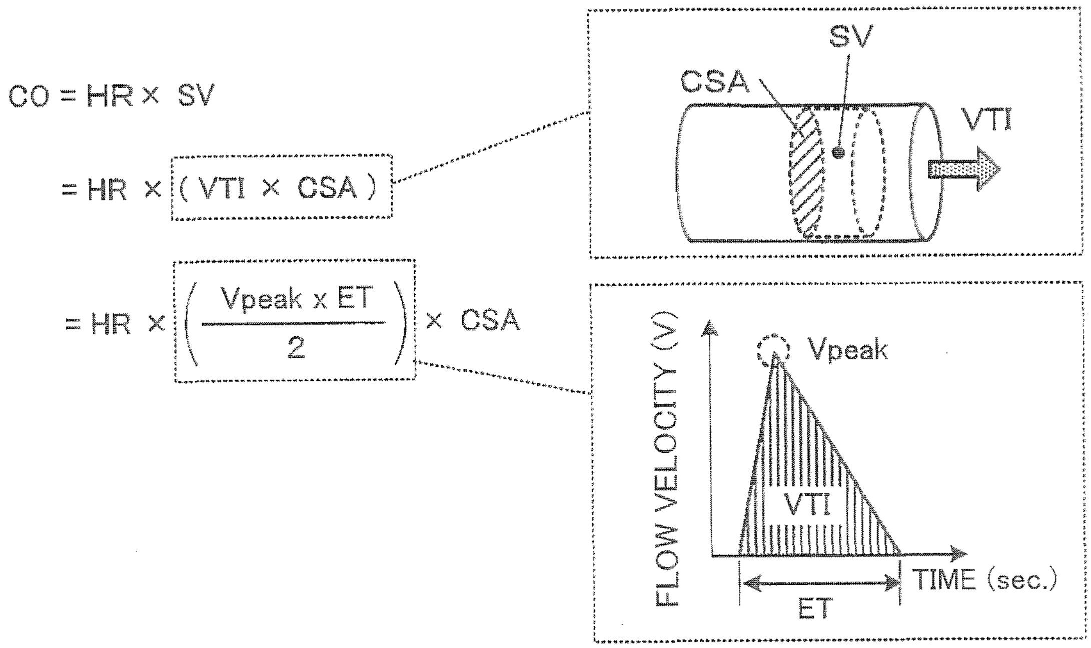
CO = HR × SV
= HR × ( VTI × CSA )
= HR × ( Vpeak x ET / 2 ) × CSA
SV
CSA
VTI
FLOW VELOCITY (V)
Vpeak
VTI
TIME (sec.)
ET

FIG. 7

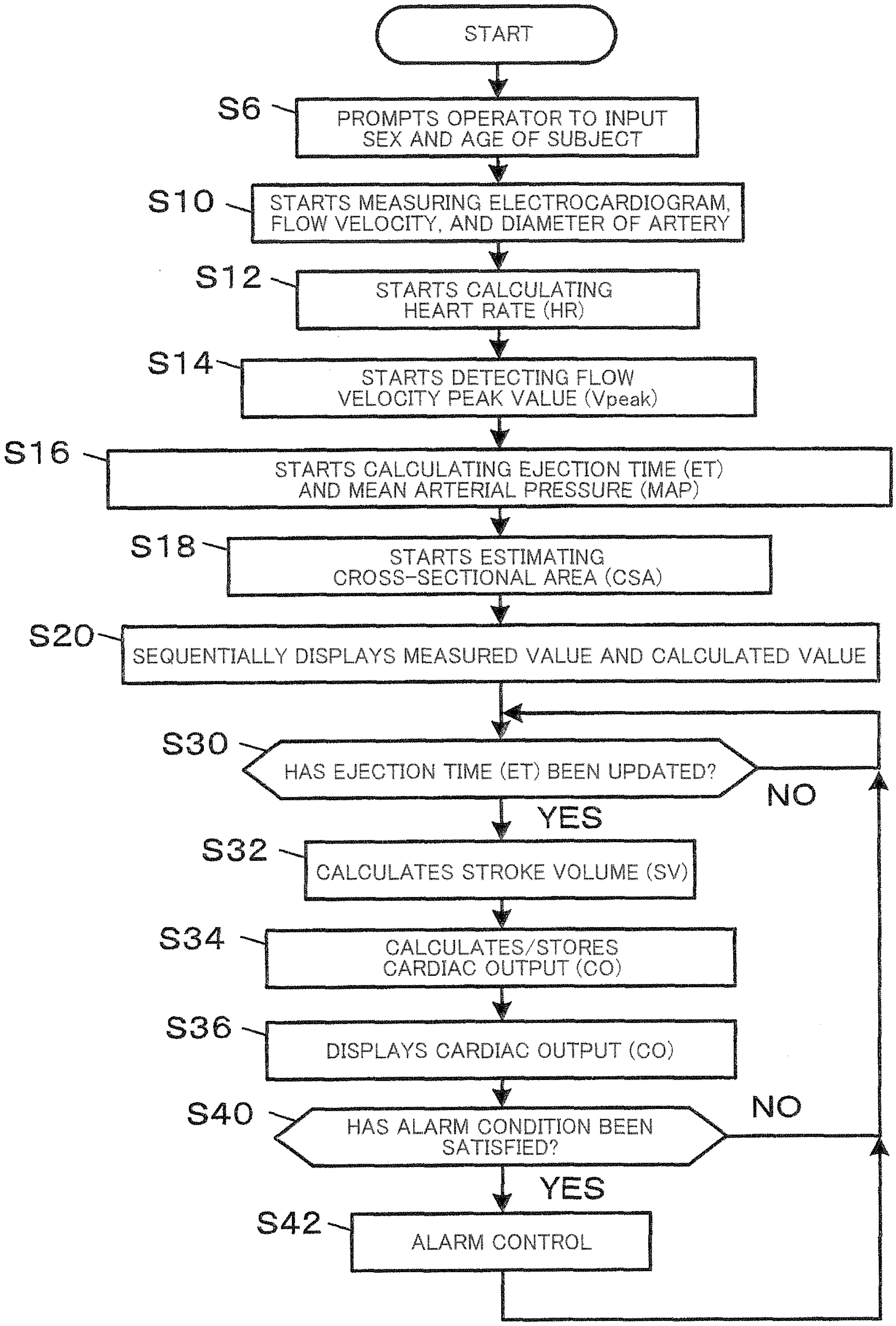
START
S6
PROMPTS OPERATOR TO INPUT SEX AND AGE OF SUBJECT
S10
STARTS MEASURING ELECTROCARDIOGRAM, FLOW VELOCITY, AND DIAMETER OF ARTERY
S12
STARTS CALCULATING HEART RATE (HR)
S14
STARTS DETECTING FLOW VELOCITY PEAK VALUE (Vpeak)
S16
STARTS CALCULATING EJECTION TIME (ET) AND MEAN ARTERIAL PRESSURE (MAP)
S18
STARTS ESTIMATING CROSS-SECTIONAL AREA (CSA)
S20
SEQUENTIALLY DISPLAYS MEASURED VALUE AND CALCULATED VALUE
S30
HAS EJECTION TIME (ET) BEEN UPDATED?
NO
YES
S32
CALCULATES STROKE VOLUME (SV)
S34
CALCULATES/STORES CARDIAC OUTPUT (CO)
S36
DISPLAYS CARDIAC OUTPUT (CO)
S40
HAS ALARM CONDITION BEEN SATISFIED?
NO
YES
S42
ALARM CONTROL

FIG. 8

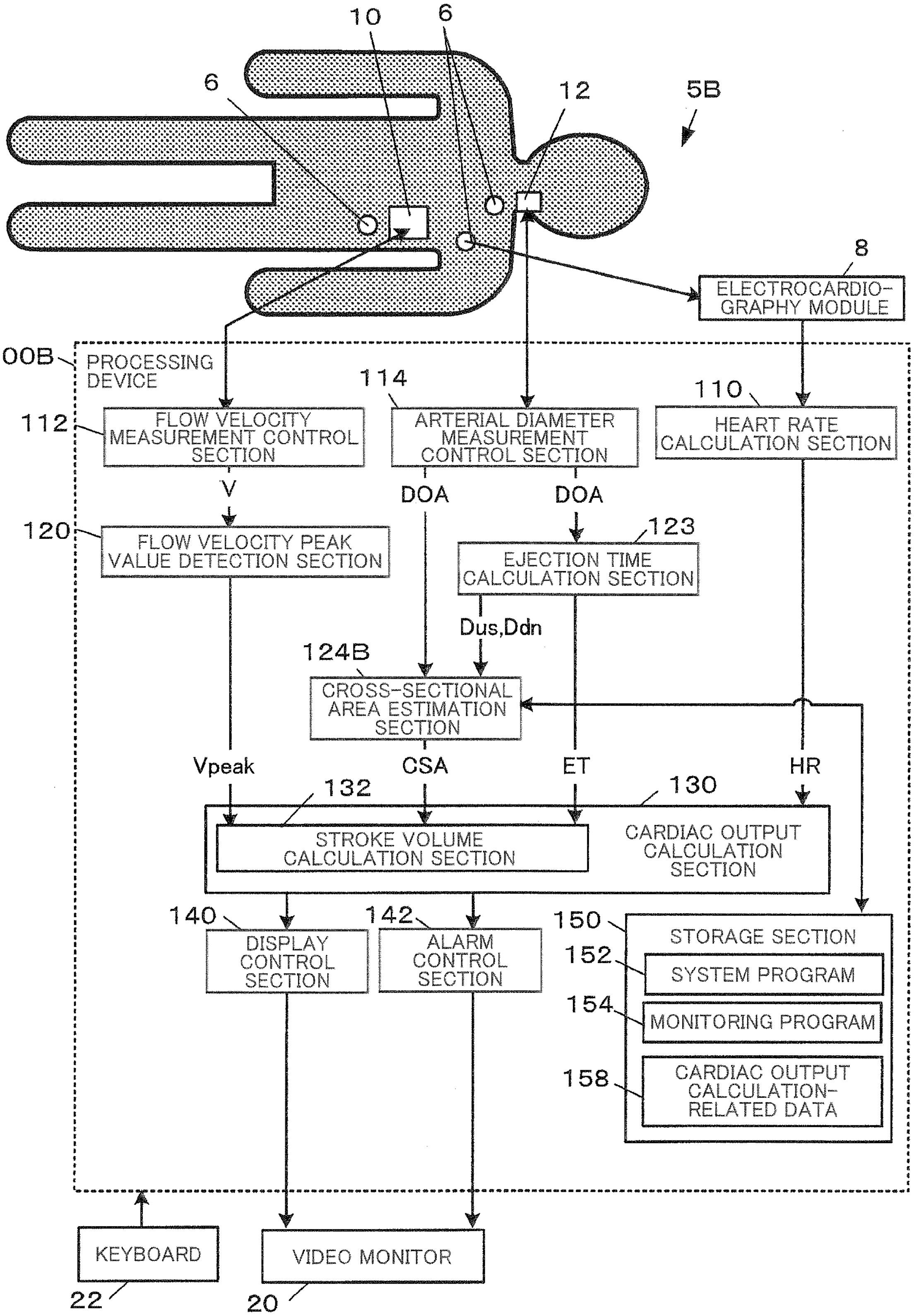
10
6
6
12
5B
8
ELECTROCARDIOGRAPHY MODULE
100B
PROCESSING DEVICE
114
110
112
FLOW VELOCITY MEASUREMENT CONTROL SECTION
ARTERIAL DIAMETER MEASUREMENT CONTROL SECTION
HEART RATE CALCULATION SECTION
V
DOA
DOA
120
FLOW VELOCITY PEAK VALUE DETECTION SECTION
123
EJECTION TIME CALCULATION SECTION
Dus,Ddn
124B
CROSS-SECTIONAL AREA ESTIMATION SECTION
Vpeak
CSA
ET
HR
132
130
STROKE VOLUME CALCULATION SECTION
CARDIAC OUTPUT CALCULATION SECTION
140
142
150
DISPLAY CONTROL SECTION
ALARM CONTROL SECTION
STORAGE SECTION
152
SYSTEM PROGRAM
154
MONITORING PROGRAM
158
CARDIAC OUTPUT CALCULATION-RELATED DATA
KEYBOARD
VIDEO MONITOR
22
20

FIG. 9

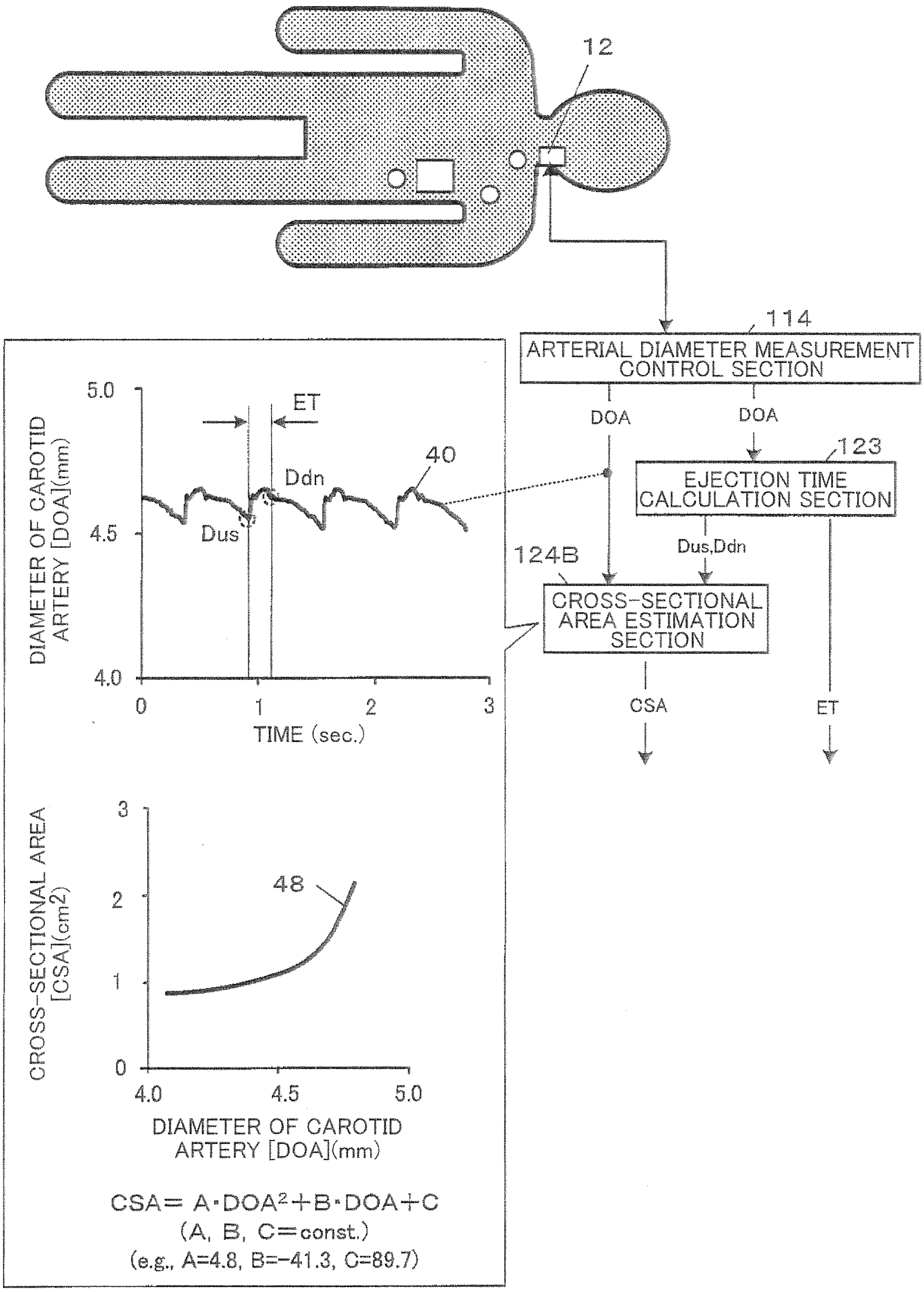
12
114
ARTERIAL DIAMETER MEASUREMENT CONTROL SECTION
DOA
DOA
123
EJECTION TIME CALCULATION SECTION
Dus,Ddn
124B
CROSS-SECTIONAL AREA ESTIMATION SECTION
CSA
ET
DIAMETER OF CAROTID ARTERY [DOA](mm)
5.0
4.5
4.0
ET
Ddn
Dus
40
0
1
2
3
TIME (sec.)
CROSS-SECTIONAL AREA [CSA](cm2)
3
2
1
0
48
4.0
4.5
5.0
DIAMETER OF CAROTID ARTERY [DOA](mm)
CSA= A·DOA²+B·DOA+C
(A, B, C=const.)
(e.g., A=4.8, B=−41.3, C=89.7)

FIG. 10

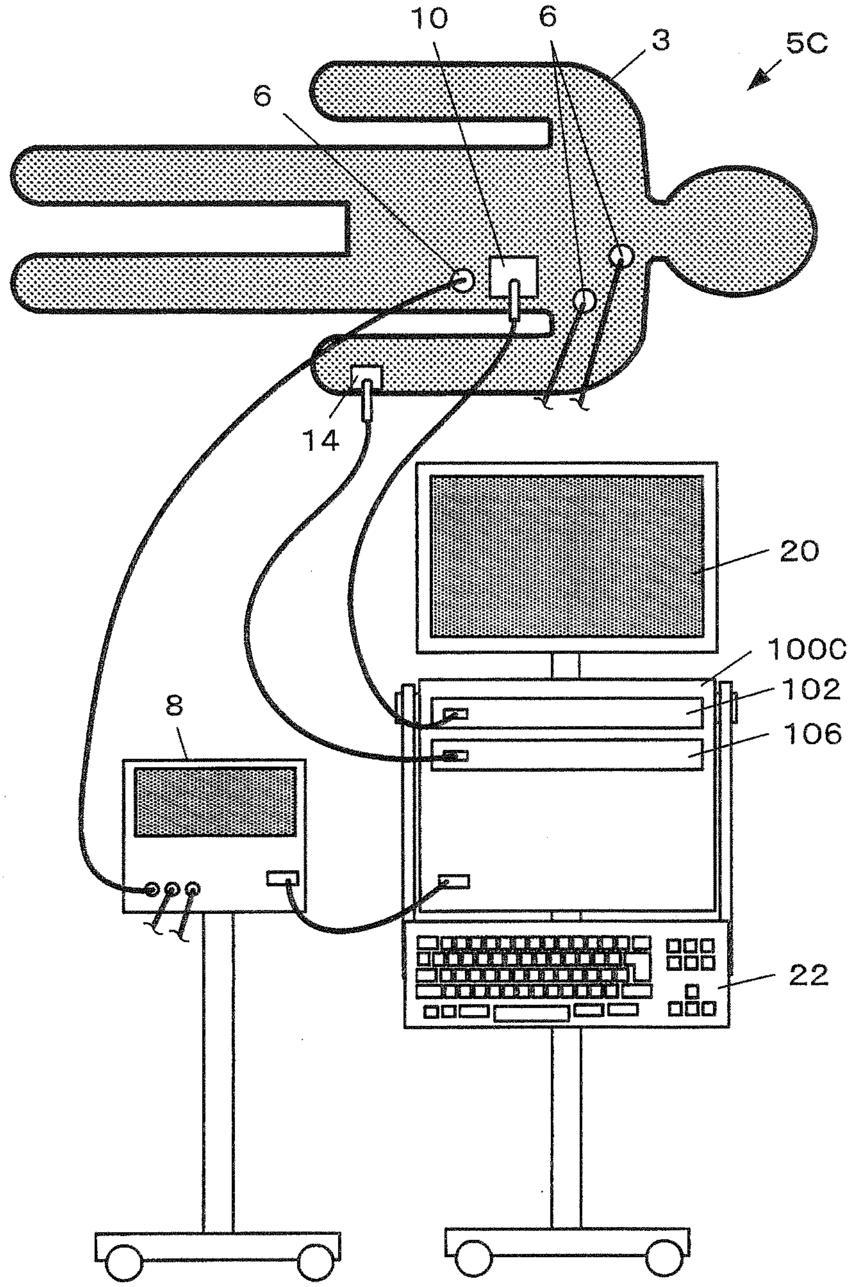

FIG. 11

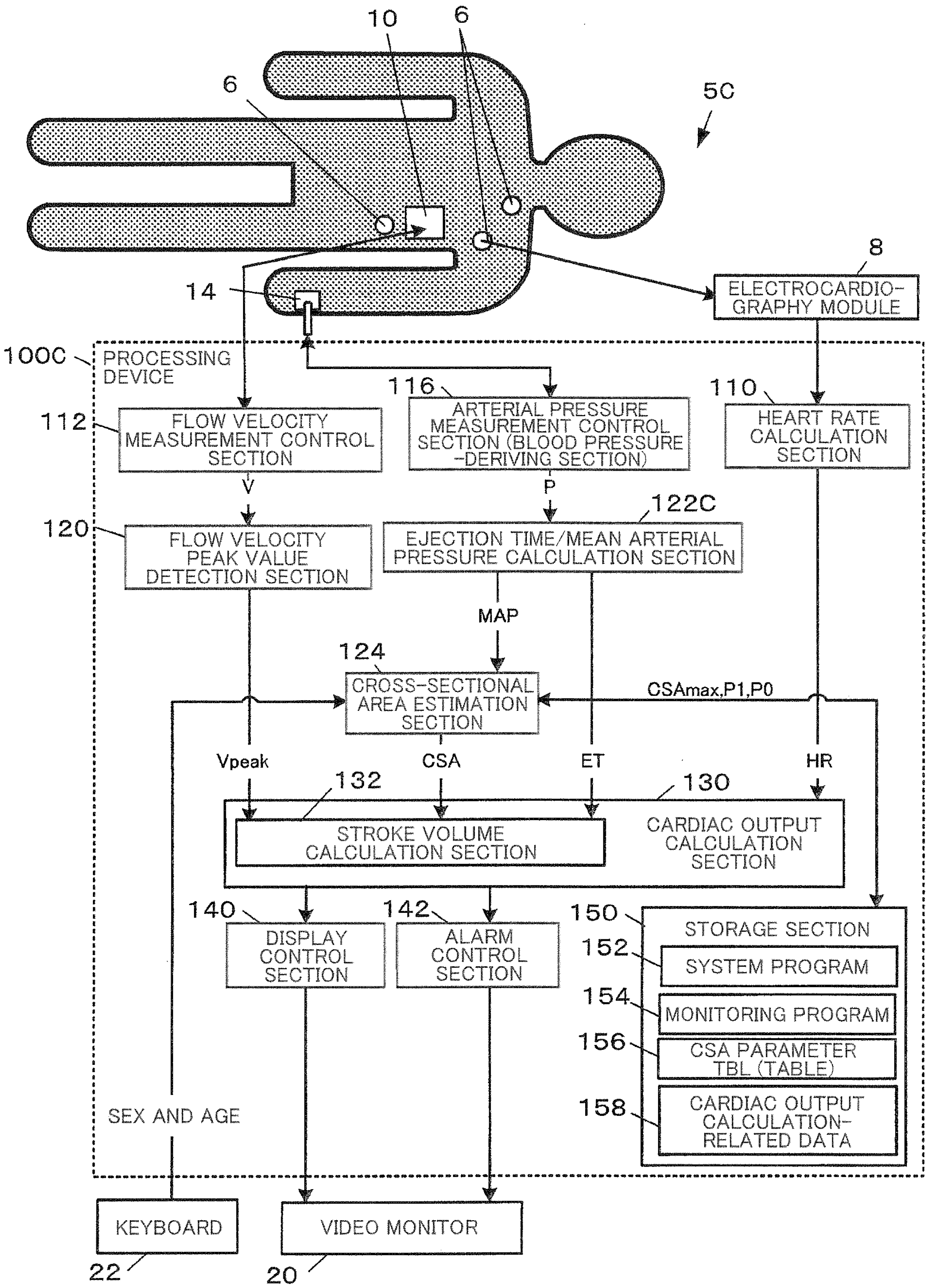
10
6
6
5C
8
ELECTROCARDIOGRAPHY MODULE
14
100C
PROCESSING DEVICE
116
ARTERIAL PRESSURE MEASUREMENT CONTROL SECTION (BLOOD PRESSURE -DERIVING SECTION)
110
HEART RATE CALCULATION SECTION
112
FLOW VELOCITY MEASUREMENT CONTROL SECTION
V
P
122C
120
FLOW VELOCITY PEAK VALUE DETECTION SECTION
EJECTION TIME/MEAN ARTERIAL PRESSURE CALCULATION SECTION
MAP
124
CROSS-SECTIONAL AREA ESTIMATION SECTION
CSAmax,P1,P0
Vpeak
CSA
ET
HR
132
130
STROKE VOLUME CALCULATION SECTION
CARDIAC OUTPUT CALCULATION SECTION
140
DISPLAY CONTROL SECTION
142
ALARM CONTROL SECTION
150
STORAGE SECTION
152
SYSTEM PROGRAM
154
MONITORING PROGRAM
156
CSA PARAMETER TBL (TABLE)
158
CARDIAC OUTPUT CALCULATION-RELATED DATA
SEX AND AGE
KEYBOARD
22
VIDEO MONITOR
20

FIG. 12

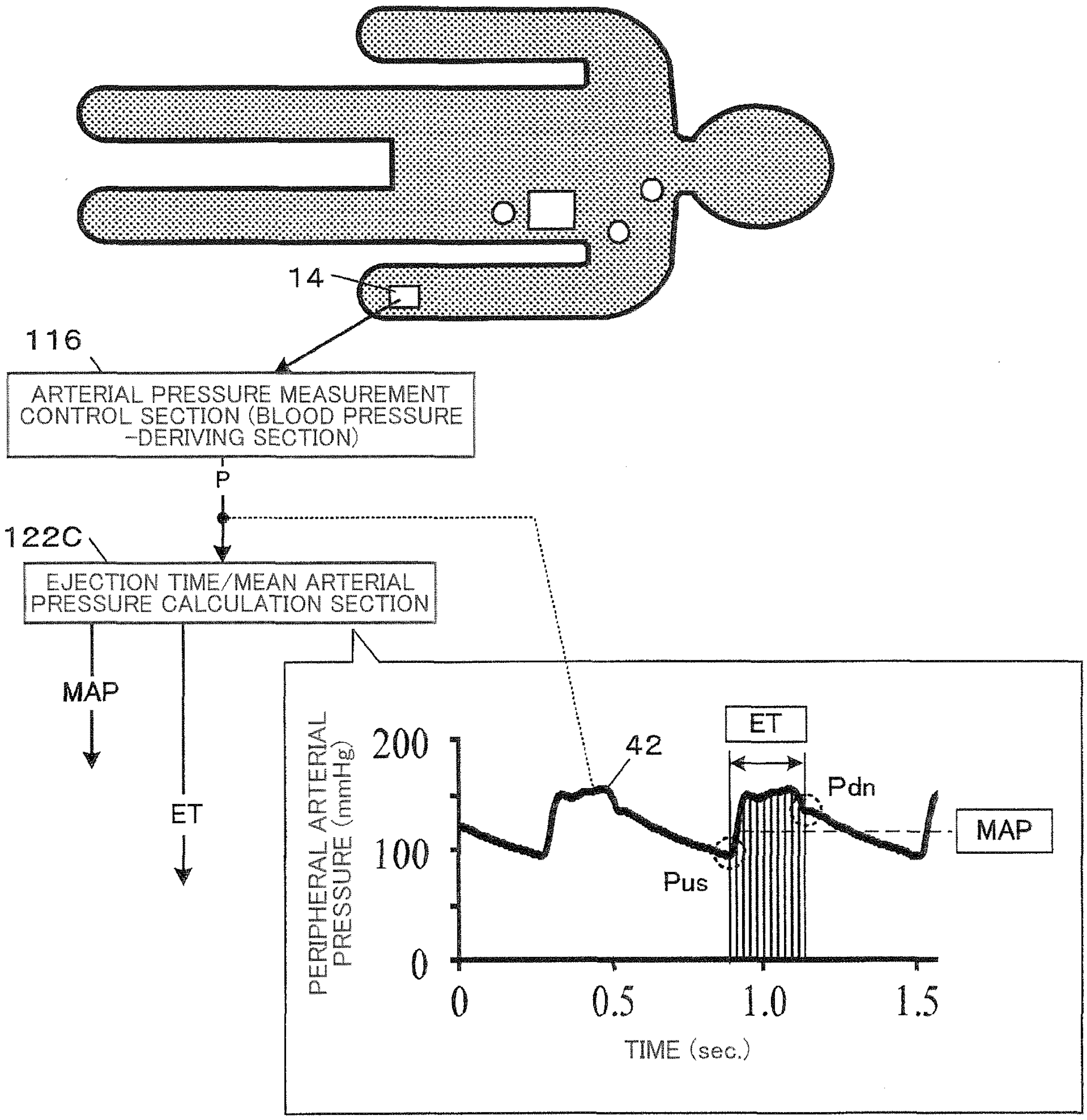
14
116
ARTERIAL PRESSURE MEASUREMENT CONTROL SECTION (BLOOD PRESSURE -DERIVING SECTION)
P
122C
EJECTION TIME/MEAN ARTERIAL PRESSURE CALCULATION SECTION
MAP
ET
PERIPHERAL ARTERIAL PRESSURE (mmHg)
200
100
0
0.5
1.0
1.5
TIME (sec.)
42
ET
Pdn
Pus
MAP

FIG. 13

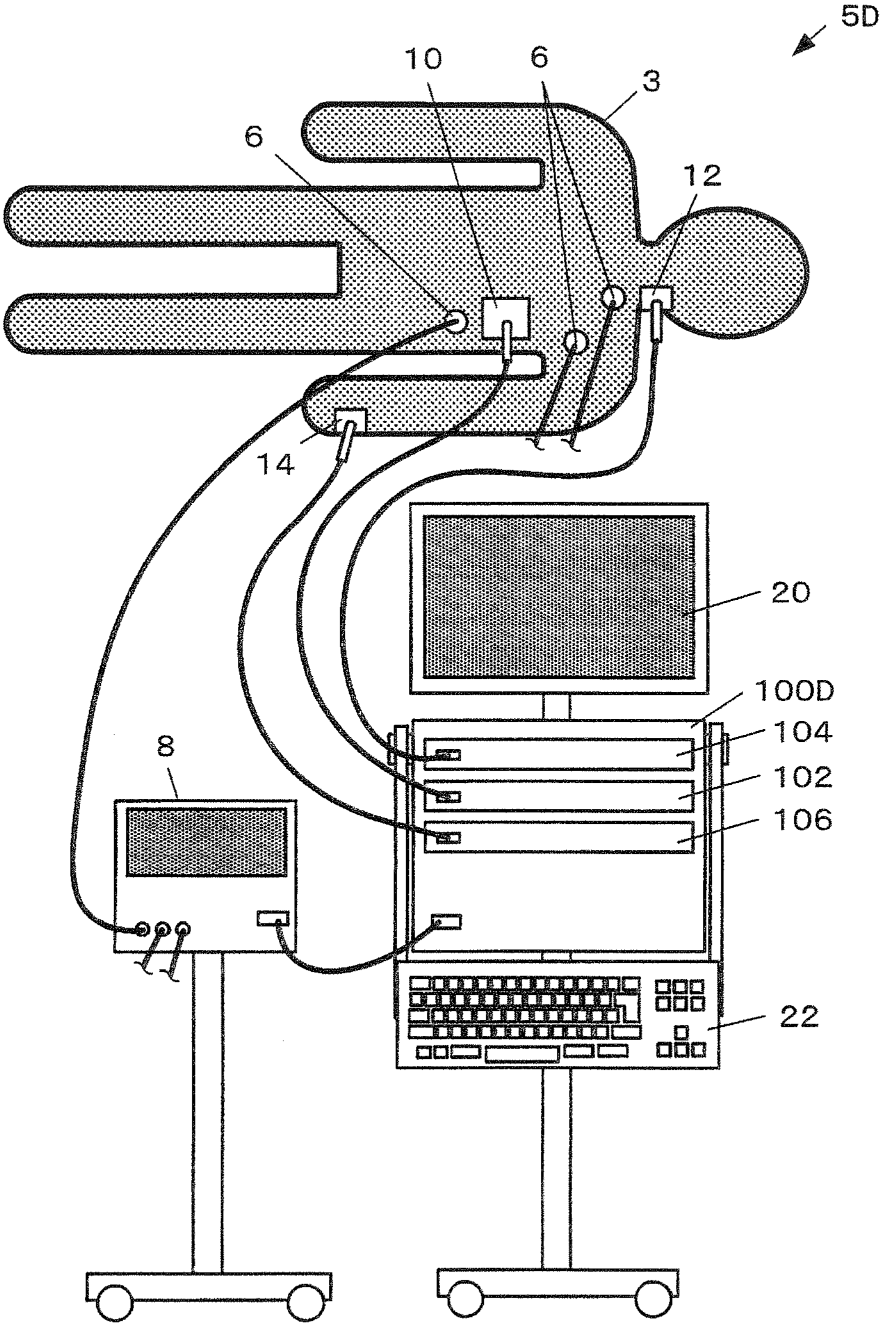

FIG. 14

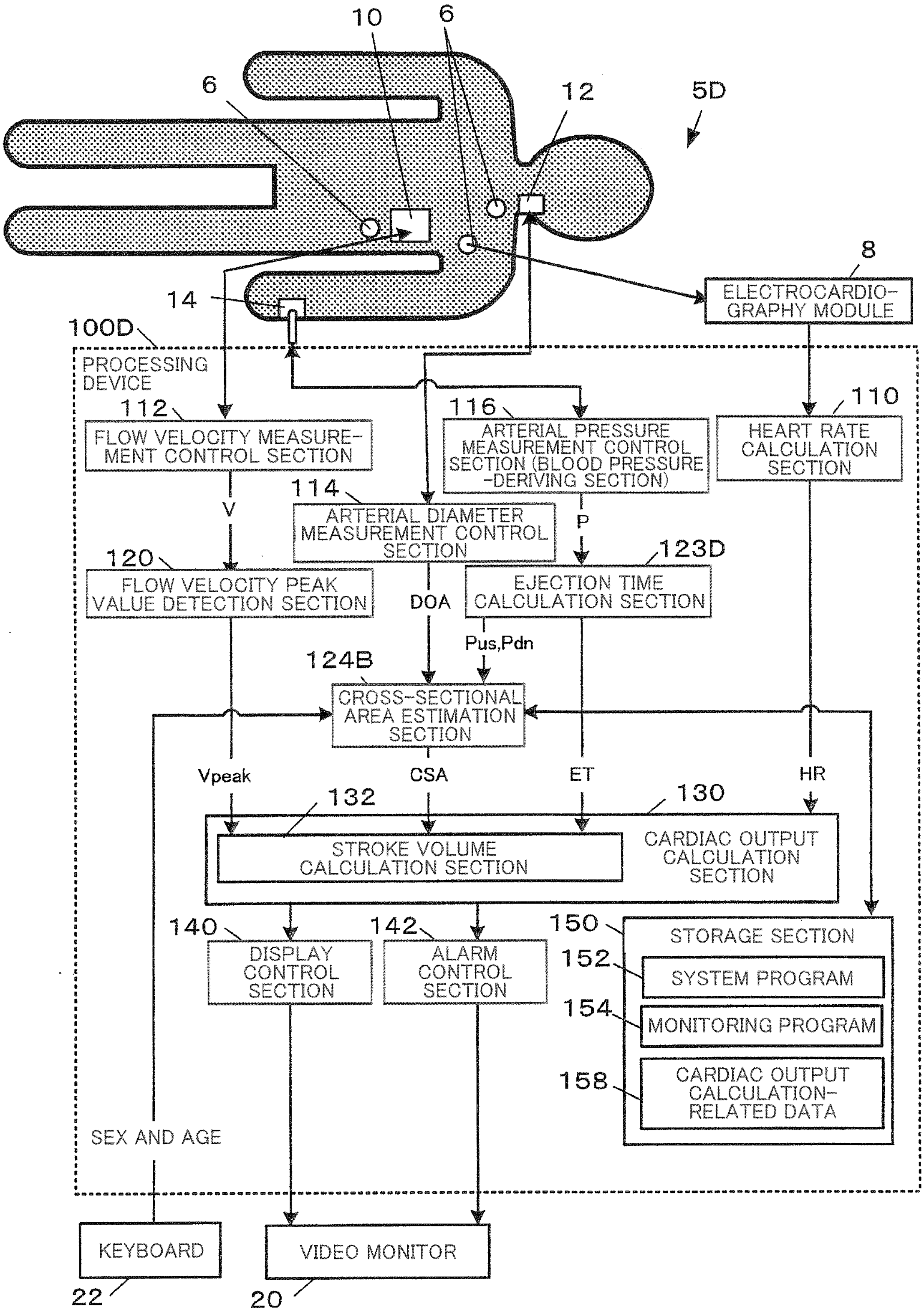
10
6
6
12
5D
8
14
100D
ELECTROCARDIOGRAPHY MODULE
PROCESSING DEVICE
112
FLOW VELOCITY MEASUREMENT CONTROL SECTION
116
ARTERIAL PRESSURE MEASUREMENT CONTROL SECTION (BLOOD PRESSURE-DERIVING SECTION)
110
HEART RATE CALCULATION SECTION
V
114
ARTERIAL DIAMETER MEASUREMENT CONTROL SECTION
P
120
FLOW VELOCITY PEAK VALUE DETECTION SECTION
DOA
123D
EJECTION TIME CALCULATION SECTION
Pus,Pdn
124B
CROSS-SECTIONAL AREA ESTIMATION SECTION
Vpeak
CSA
ET
HR
132
130
STROKE VOLUME CALCULATION SECTION
CARDIAC OUTPUT CALCULATION SECTION
140
DISPLAY CONTROL SECTION
142
ALARM CONTROL SECTION
150
STORAGE SECTION
152
SYSTEM PROGRAM
154
MONITORING PROGRAM
158
CARDIAC OUTPUT CALCULATION-RELATED DATA
SEX AND AGE
KEYBOARD
22
VIDEO MONITOR
20

FIG. 15

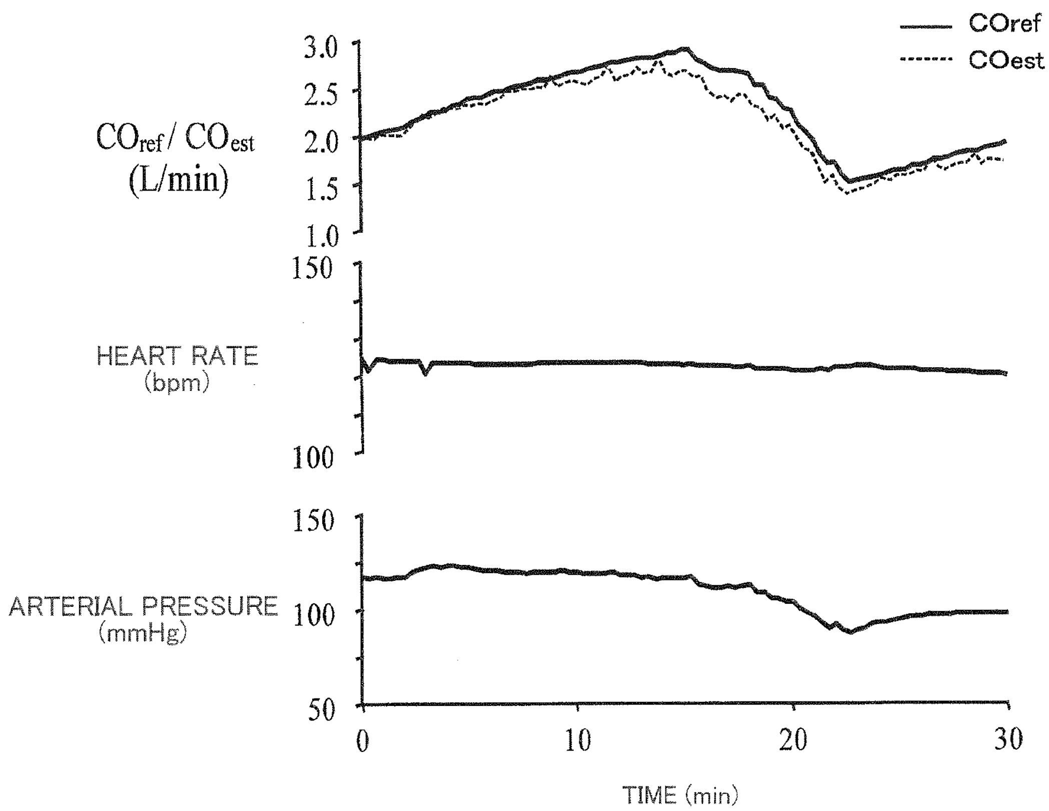
COref
COest
3.0
2.5
2.0
1.5
1.0
$CO_{ref}$ / $CO_{est}$
(L/min)
150
HEART RATE
(bpm)
100
150
ARTERIAL PRESSURE
(mmHg)
100
50
0
10
20
30
TIME (min)

FIG. 16

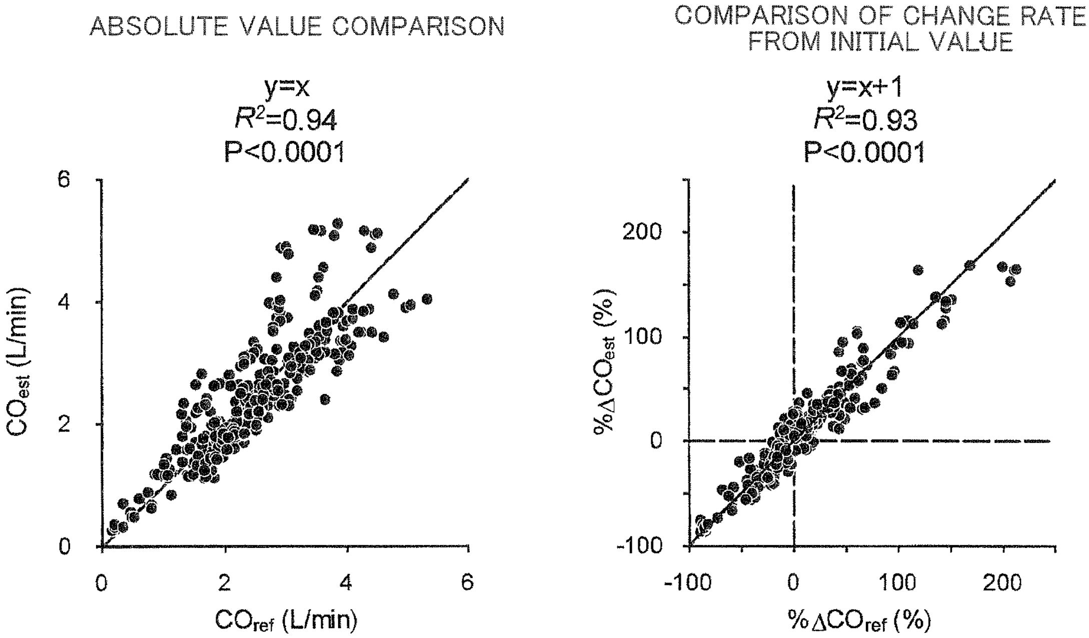
ABSOLUTE VALUE COMPARISON
y=x
$R^2$=0.94
P<0.0001
$CO_{est}$ (L/min)
$CO_{ref}$ (L/min)
0
2
4
6
COMPARISON OF CHANGE RATE FROM INITIAL VALUE
y=x+1
$R^2$=0.93
P<0.0001
%$\Delta CO_{est}$ (%)
%$\Delta CO_{ref}$ (%)
-100
0
100
200

FIG. 17

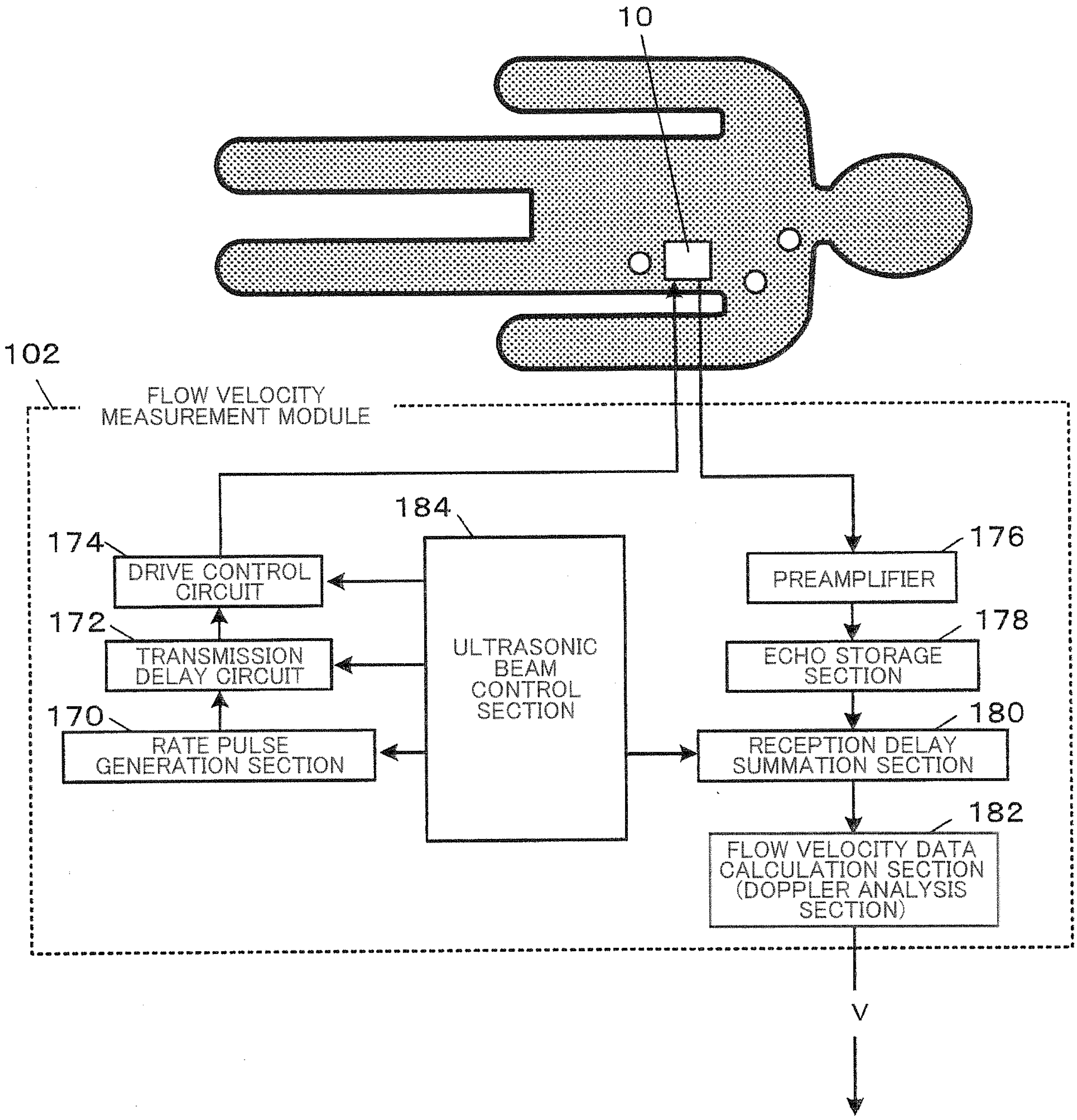
10
102
FLOW VELOCITY MEASUREMENT MODULE
184
174
DRIVE CONTROL CIRCUIT
172
TRANSMISSION DELAY CIRCUIT
170
RATE PULSE GENERATION SECTION
ULTRASONIC BEAM CONTROL SECTION
176
PREAMPLIFIER
178
ECHO STORAGE SECTION
180
RECEPTION DELAY SUMMATION SECTION
182
FLOW VELOCITY DATA CALCULATION SECTION (DOPPLER ANALYSIS SECTION)
V

FIG. 18

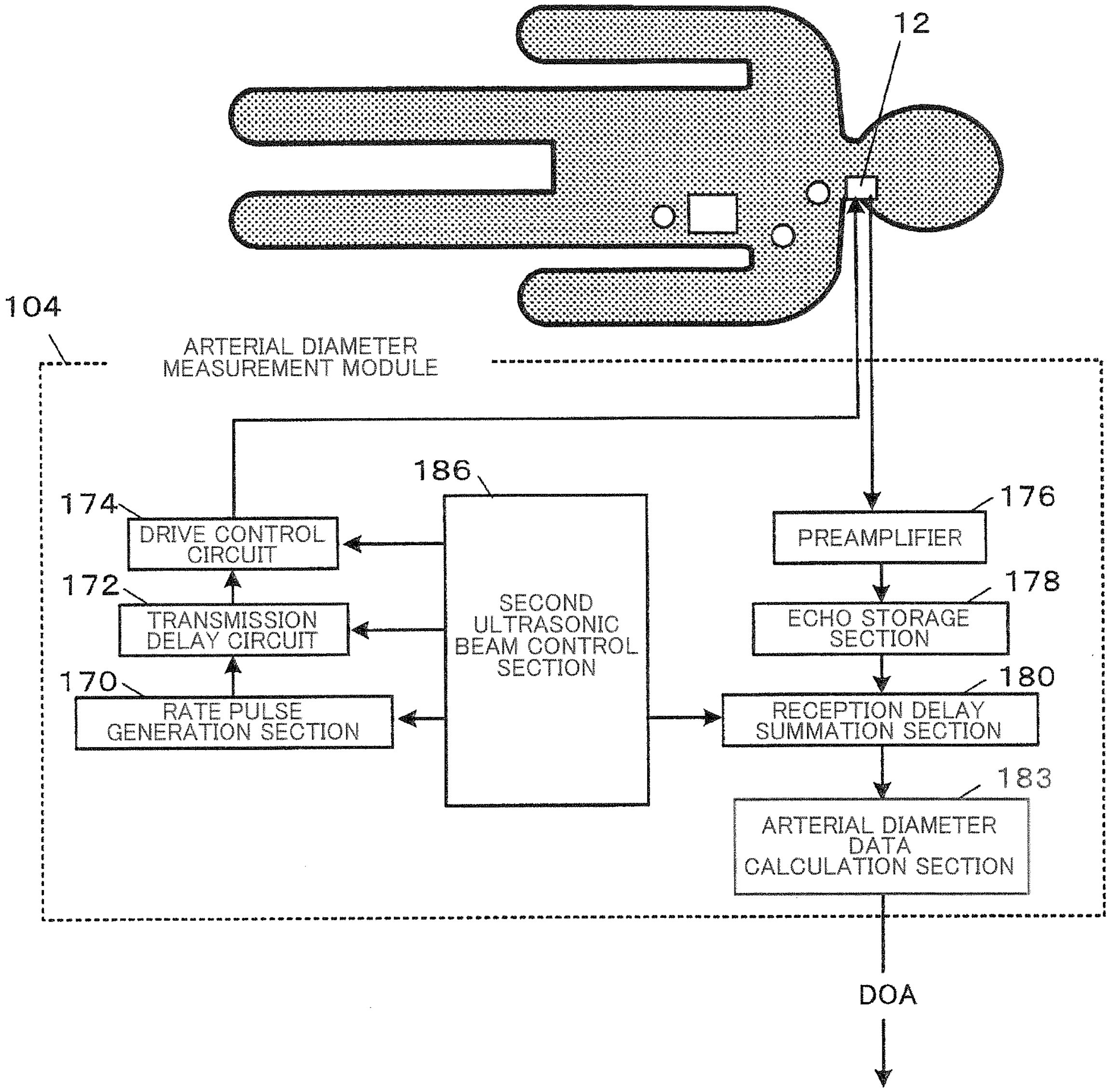
12
104
ARTERIAL DIAMETER MEASUREMENT MODULE
186
174
DRIVE CONTROL CIRCUIT
172
TRANSMISSION DELAY CIRCUIT
170
RATE PULSE GENERATION SECTION
SECOND ULTRASONIC BEAM CONTROL SECTION
176
PREAMPLIFIER
178
ECHO STORAGE SECTION
180
RECEPTION DELAY SUMMATION SECTION
183
ARTERIAL DIAMETER DATA CALCULATION SECTION
DOA

FIG. 19

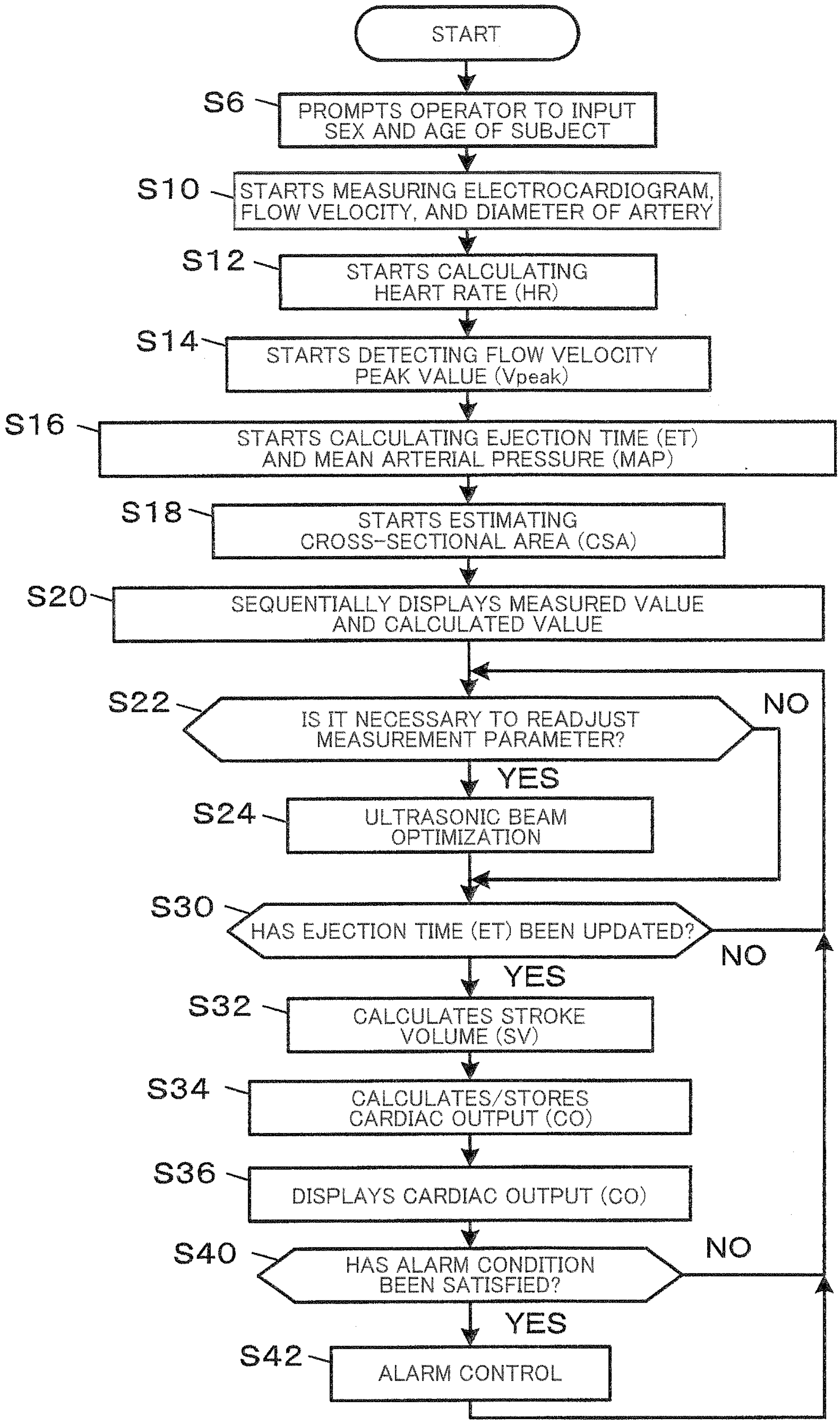
START
S6
PROMPTS OPERATOR TO INPUT SEX AND AGE OF SUBJECT
S10
STARTS MEASURING ELECTROCARDIOGRAM, FLOW VELOCITY, AND DIAMETER OF ARTERY
S12
STARTS CALCULATING HEART RATE (HR)
S14
STARTS DETECTING FLOW VELOCITY PEAK VALUE (Vpeak)
S16
STARTS CALCULATING EJECTION TIME (ET) AND MEAN ARTERIAL PRESSURE (MAP)
S18
STARTS ESTIMATING CROSS-SECTIONAL AREA (CSA)
S20
SEQUENTIALLY DISPLAYS MEASURED VALUE AND CALCULATED VALUE
S22
IS IT NECESSARY TO READJUST MEASUREMENT PARAMETER?
NO
YES
S24
ULTRASONIC BEAM OPTIMIZATION
S30
HAS EJECTION TIME (ET) BEEN UPDATED?
NO
YES
S32
CALCULATES STROKE VOLUME (SV)
S34
CALCULATES/STORES CARDIAC OUTPUT (CO)
S36
DISPLAYS CARDIAC OUTPUT (CO)
S40
HAS ALARM CONDITION BEEN SATISFIED?
NO
YES
S42
ALARM CONTROL

FIG. 20

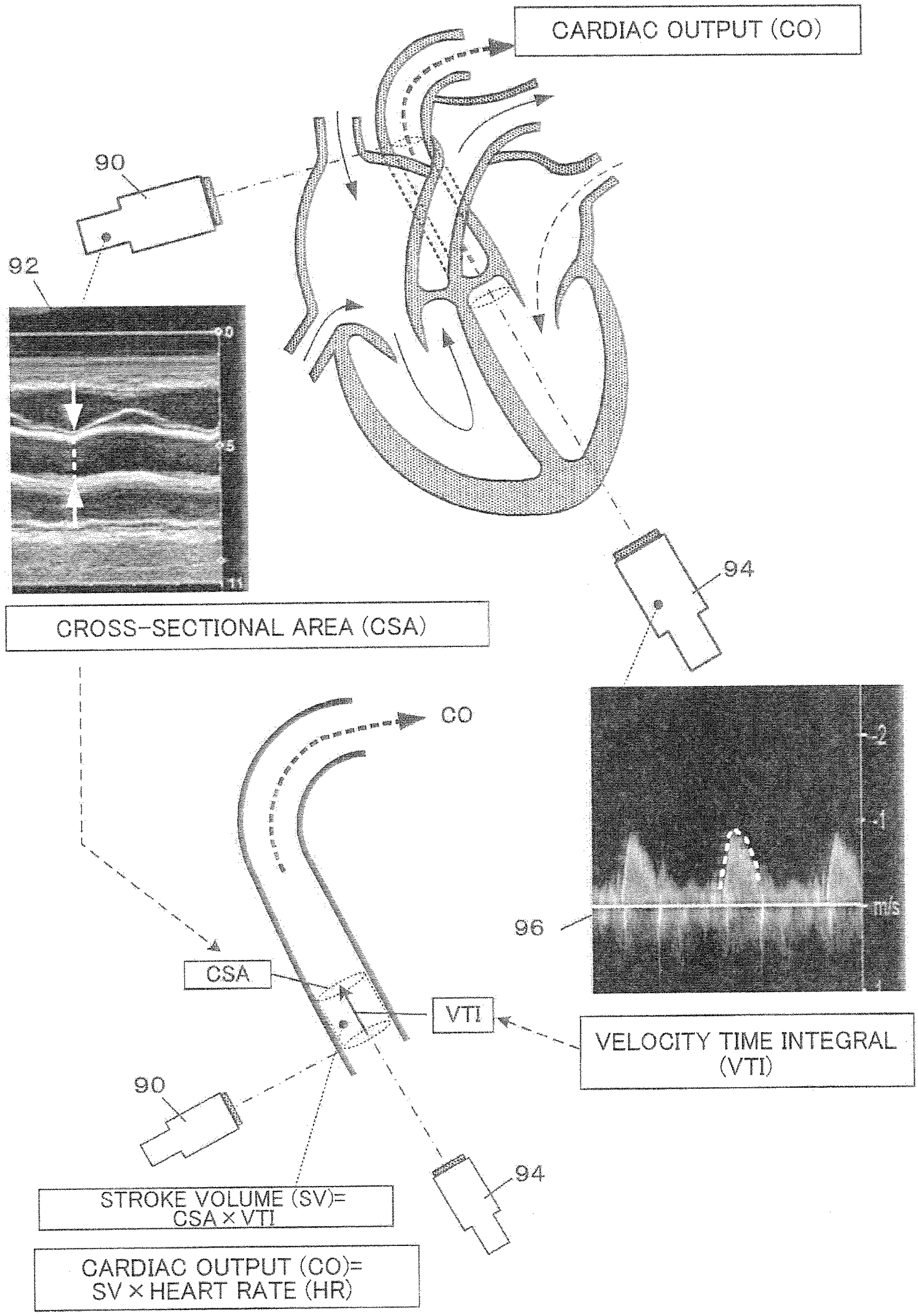
CARDIAC OUTPUT (CO)
90
92
CROSS-SECTIONAL AREA (CSA)
94
CO
96
CSA
VTI
VELOCITY TIME INTEGRAL (VTI)
90
94
STROKE VOLUME (SV)= CSA × VTI
CARDIAC OUTPUT (CO)= SV × HEART RATE (HR)

Europäisches Patentamt
European Patent Office
Office européen des brevets

# EUROPEAN SEARCH REPORT

Application Number
EP 13 16 6623

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/185084 A1 (ZHANG HONGXUAN [US]) 22 July 2010 (2010-07-22)<br>* paragraphs [0007], [0015] - [0035] *<br>----- | 1-10 | INV.<br>A61B8/06<br>A61B5/029 |
| A | US 2007/197924 A1 (O'ROURKE MICHAEL F [AU]) 23 August 2007 (2007-08-23)<br>* the whole document *<br>----- | 1-12 | |
| A | WO 2004/002305 A2 (ALLEZ PHYSIONIX [CA]; UNIV WASHINGTON [US]; MOURAD PIERRE [US]; KLIOT) 8 January 2004 (2004-01-08)<br>* page 10, line 23 - page 14, line 5 *<br>* page 40, line 31 - page 41, line 30 *<br>----- | 1-12 | |

TECHNICAL FIELDS SEARCHED (IPC)

A61B

The present search report has been drawn up for all claims

1

| Place of search | Date of completion of the search | Examiner |
|---|---|---|
| Berlin | 29 August 2013 | Pohjamo, Terhi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..............................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 16 6623

This annex lists the patent family members relating to the patent documents cited in the above-mentioned European search report.
The members are as contained in the European Patent Office EDP file on
The European Patent Office is in no way liable for these particulars which are merely given for the purpose of information.

29-08-2013

| Patent document cited in search report | Publication date | Patent family member(s) | Publication date |
|---|---|---|---|
| US 2010185084 A1 | 22-07-2010 | NONE | |
| US 2007197924 A1 | 23-08-2007 | EP 1722679 A1 | 22-11-2006 |
| | | JP 2007526040 A | 13-09-2007 |
| | | US 2007197924 A1 | 23-08-2007 |
| | | WO 2005084536 A1 | 15-09-2005 |
| WO 2004002305 A2 | 08-01-2004 | AU 2003280416 A1 | 19-01-2004 |
| | | CA 2490999 A1 | 08-01-2004 |
| | | EP 1531725 A2 | 25-05-2005 |
| | | JP 2005532097 A | 27-10-2005 |
| | | WO 2004002305 A2 | 08-01-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 10094528 A


### Non-patent literature cited in the description


- *Journal of Applied Physiology,* 01 May 1993, vol. 74 (5), 2566
- **DONNERSTEIN et al.** Simplified method for estimation of Doppler cardiac output in the great arteries. *The American Journal of Cardiology,* July 1988, vol. 62 (1), 155-156